(19) Europäisches Patentamt / European Patent Office / Office européen des brevets

(11) **EP 3 644 852 B1**

(12) **EUROPÄISCHE PATENTSCHRIFT**

(45) Veröffentlichungstag und Bekanntmachung des Hinweises auf die Patenterteilung:
**12.01.2022 Patentblatt 2022/02**

(21) Anmeldenummer: **18793163.9**

(22) Anmeldetag: **27.06.2018**

(51) Int Cl.:
*A61B 5/107* *(2006.01)*     *A61B 5/11* *(2006.01)*
*A61G 7/00* *(2006.01)*     *A61H 1/02* *(2006.01)*

(86) Internationale Anmeldenummer:
**PCT/DE2018/100587**

(87) Internationale Veröffentlichungsnummer:
**WO 2019/001636 (03.01.2019 Gazette 2019/01)**

(54) **MESSVERFAHREN UND VORRICHTUNG ZUR BESTIMMUNG DER LÄNGENVERHÄLTNISSE, DER POSITION UND/ODER DES BEWEGUNGSRADIUS DER UNTEREN EXTREMITÄTEN EINES BETTPFLICHTIGEN PATIENTEN**

MEASURING METHOD AND APPARATUS FOR DETERMINING THE LENGTH CONDITIONS, THE POSITION AND/OR THE RADIUS OF MOVEMENT OF THE LOWER EXTREMITIES OF A BED-BOUND PATIENT

PROCÉDÉ DE MESURE ET DISPOSITIF PERMETTANT DE DÉTERMINER LES RAPPORTS DE LONGUEUR, LA POSITION ET/OU LE RAYON DE DÉPLACEMENT DES EXTRÉMITÉS INFÉRIEURES D'UN PATIENT ALITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.06.2017 DE 102017114290**

(43) Veröffentlichungstag der Anmeldung:
**06.05.2020 Patentblatt 2020/19**

(73) Patentinhaber: **ReActive Robotics GmbH**
**80687 München (DE)**

(72) Erfinder:
• **PEYRL, Helfried**
**82041 Oberhaching (DE)**
• **ZILLIG, Oliver**
**80801 München (DE)**
• **JONES, Kathleen**
**80333 München (DE)**
• **METZNER, Philipp**
**85354 Freising (DE)**

(74) Vertreter: **Heilein, Ernst-Peter et al**
**HEILEIN IP LAW**
**Bezirksstraße 2**
**85716 Unterschleißheim (DE)**

(56) Entgegenhaltungen:
CN-A- 106 621 207     DE-B3-102015 117 596
JP-A- H1 176 329

**Beschreibung**

Die vorliegende Erfindung betrifft ein Messverfahren zur Bestimmung der Längenverhältnisse, der Position und/oder des Bewegungsradius der unteren Extremitäten eines bettpflichtigen Patienten, wie Hüftgelenk, Kniegelenk und/oder Oberschenkel, sowie eine Vorrichtung zur Durchführung eines solchen Verfahrens, wenigstens umfassend einen Rehabilitationsmechanismus mit wenigstens einem in Wirkverbindung mit dem Kniegelenk eines Patienten bringbaren Kniemodul, umfassend wenigstens einen Elektromotor zum Antrieb von wenigstens zwei Exzentern.

Bettpflichtige Patienten - im Folgenden werden darunter insbesondere Patienten verstanden, die, ob bei Bewusstsein oder bewusstlos, aus medizinischen Gründen ein Bett aus eigener Kraft für eine längere Zeit nicht verlassen können - profitieren nachweislich von einer regelmäßig durchgeführten Physiotherapie.

Physiotherapeutische Übungen, die der Patient meist mit Hilfe einer physiotherapeutisch ausgebildeten Fachkraft durchführt, können während einer längeren Phase der Bettlägerigkeit die Beweglichkeit und Muskelmasse des Patienten erhalten, die Durchblutung des Körpers fördern und so beispielsweise auch das Thromboserisiko senken.

Bei bewusstlosen bettpflichtigen Patienten hat sich dabei insbesondere eine Bewegungstherapie der unteren Extremitäten als effektiv erwiesen, da durch eine Bewegung der Beine große Muskelgruppen des Körpers trainiert werden und so ein größerer Stimulus für das Gehirn des Patienten ausgelöst werden kann. Physiotherapeutische Übungen können dadurch sogar die Genesung verletzter Hirnareale beschleunigen und das Erwachen aus dem Koma erleichtern.

Einem großen medizinischen Nutzen der Bewegungstherapie steht allerdings auch ein großer personeller Aufwand und eine damit einhergehende entsprechend große finanzielle Belastung der Krankenhäuser gegenüber, die oftmals innerhalb des gesetzlichen Gesundheitssystems nicht geleistet werden kann. Gerade Komapatienten leiden dann unter einer zu geringen Übungsanzahl und Übungsdauer.

Aus diesem Grund wird in den letzten Jahren versucht, die klassisch von Physiotherapeuten manuell durchgeführte Bewegungstherapie von bettpflichtigen Patienten mehr und mehr zu automatisieren.

Aus den Druckschriften JP 2005 334385 A, JP H10 258101 A, WO 00/61059 A1, JP 2003 225264 A, CN 106 621 207 A, sowie aus der DE 10 2015 117 596 B3 bzw. der WO 2017/063639 A1 der Anmelderin sind beispielsweise verschiedene Rehabilitationsmechanismen bekannt, mit deren Hilfe physiotherapeutische Übungen, insbesondere eine Gangtherapie, automatisiert durchgeführt werden können.

In diesem Zusammenhang wird in der JP H11 76329 A eine Vorrichtung zur automatisierten Bewegung der Gliedmaßen einer Person offenbart, welche aus Sensordaten mittels einer Messeinrichtung für Proportionsdaten Informationen bezüglich der Körpermaße eines Benutzers bestimmt und daraus dann mittels einer Steuereinrichtung die Trajektorie für die Vorrichtung zur automatisierten Bewegung der Gliedmaßen berechnet und als Steuerbefehle an die Vorrichtung weitergibt. Dazu wird aus den Längen und zueinander eingenommenen Winkeln der Hebelarme der Vorrichtung zum einen die Länge zwischen dem Hüftgelenk eines Benutzers und einem Aufnahmemittel für eine Gliedmaße des Benutzers, und zum anderen der Abstand zwischen dem Hüftgelenk und einem Nullpunkt des Vorrichtungskoordinatensystems auf der Basis der Vorrichtung bestimmt. Auf Basis dieser Daten wird dann die durch die Vorrichtung bei einer automatisierten Bewegung abzufahrenden Trajektorie berechnet. Die hier offenbarte Vorrichtung kann sich somit vorteilhaft selbst bezüglich der individuellen Anatomie des jeweiligen Benutzers justieren.

Im Zuge einer derartigen Bewegungstherapie aber auch für andere medizinische Untersuchungs- und/oder Therapiemethoden ist es im Krankenhausalltag immer wieder notwendig, die individuellen Längenverhältnisse, Positionen einzelner Gelenke sowie den Bewegungsradius des jeweiligen Patienten zu messen, um die verwendeten Untersuchungs- und/oder Therapiegeräte mit Hilfe der gewonnen Daten auf den jeweiligen Patienten einzustellen.

Normalerweise werden solche Messungen manuell mit einem Maßband oder beispielsweise durch ein Gerät zur Umfangs- und Längenmessung von liegenden Körperteilen einer Person, wie es aus der DE 85 14 240 U1 bekannt ist, durchgeführt, wobei oft unterschiedliche Personen, je nach Geräteverantwortlichkeit, diese Messungen durchführen. Neben einer eigentlich unnötigen Wiederholung, hat diese Praxis auch den Nachteil, dass sich die Messergebnisse allein schon aufgrund der Tatsache, dass verschiedene Personen die Messungen durchgeführt haben, voneinander unterscheiden, sodass insbesondere die Mittelwerte der Messwerte deutliche Fehler aufweisen können. Speziell wenn der Erfolg der Therapie und damit auch die Planung weiterer Therapieschritte mittels der Veränderung des Bewegungsradius des Patienten abhängig gemacht wird, ist eine Fehleranfälligkeit der Messwerte von großem Nachteil.

Um diesem Problem zu begegnen ist aus der WO 2015/127396 A1 ein Verfahren und eine Vorrichtung zur Messung der relativen Orientierung und Position zueinander benachbarter Knochen bekannt. Dieses Verfahren bzw. diese Vorrichtung zielt speziell auf eine intraoperative Bestimmung verschiedener Längen- und Orientierungsparameter der Extremitäten eines Patienten zur möglichst präzisen Positionierung orthopädischer Implantate während einer orthopädischen Operation ab. Dazu sieht dieses Verfahren die (operative) Fixierung von magnetischen und/oder optischen Sensoren und/oder Beschleunigungssensoren direkt an der Hüfte und am Oberschenkelknochen des Patienten vor. Die Fixierung der Sensoren direkt an den zu vermessenden Knochen erlaubt zwar eine relativ hohe Genauigkeit der Messparameter, stellt allerdings nachteilig durch seinen invasiven Charakter eine enorme Belastung für den jeweiligen Patienten dar und eignet sich aus diesem Grund lediglich für intra-operative Messungen und nicht, wenn keine Indikation für einen operativen Eingriff an den unteren Extremitäten des Patienten besteht, für die Gewinnung von Messwerten für eine physiotherapeutische Bewegungstherapie.

Aus der DE 100 60 466 A1 ist zudem eine Vorrichtung zur Messung der Beinbewegung und zur Bewegungs-, Umgebungs- und Untergrundsimulation bekannt, mit der eine Geh-/Laufbewegung dreidimensional erfasst und im Rahmen einer Simulation in eine virtuelle Umgebung "projiziert" werden kann.

Hiervon ausgehend liegt der vorliegenden Erfindung die Aufgabe zugrunde, ein verbessertes Messverfahren und eine verbesserte Vorrichtung zur Bestimmung der Längenverhältnisse, der Position und/oder des Bewegungsradius der unteren Extremitäten eines bettpflichtigen Patienten bereitzustellen, welches nichtinvasiv patientenspezifische Daten ermittelt, die für eine weitere, insbesondere auch automatisierte Bewegungstherapie, sowie für andere medizinische Untersuchungs- und/oder Therapiemethoden verwendet werden können.

Diese Aufgabe wird zunächst durch ein Messverfahren zur Bestimmung der Längenverhältnisse, der Position und/oder des Bewegungsradius der unteren Extremitäten eines bettpflichtigen Patienten, wie Hüftgelenk, Kniegelenk und/oder Oberschenkel, mit den Merkmalen des unabhängigen Patentanspruchs 1 gelöst sowie durch eine Vorrichtung zur Durchführung eines solchen Messverfahrens mit den Merkmales des unabhängigen Patentanspruchs 13 gelöst. Weitere vorteilhafte Ausgestaltungen und Weiterbildungen, welche einzeln oder in Kombination miteinander einsetzbar sind, sind Gegenstand der abhängigen Ansprüche.

Das erfindungsgemäße Messverfahren zeichnet sich dadurch aus, dass wenigstens ein Kniemodul eines Rehabilitationsmechanismus über einen Angriffspunkt mit einem Bein des Patienten in Wirkverbindung gebracht wird; mittels einer Messeinrichtung eine Trajektorie allein des Angriffspunkts während einer Bewegung des Beines mit dem Kniemodul des Rehabilitationsmechanismus aufgezeichnet und/oder bestimmt wird; und mittels eines Modellgenerierungsmittels aus den so erhaltenen Daten ein kinematisches Modell der Längenverhältnisse, der Position und/oder des Bewegungsradius wenigstens von Teilen der unteren Extremitäten des Patienten erstellt wird; wobei in wenigstens einem Verfahrensschritt ermittelte Koordinatenpaare $(Y_1 / Z_1, Y_2/ Z_2, ..., Y_n/ Z_n)$ oder Koordinatentripel $(Y_1 / Z_1/ t_1; Y_2 / Z_2/ t_2; .../...; Y_n / Z_n/ t_n)$ mit einer Modellfunktion in Form einer Koordinatengleichung verglichen und dadurch im Rahmen einer Kurvenanpassung ("curve-fitting") die Position $(Y_H(t) / Z_H(t))$ eines Drehpunkts (DP) und/oder die Länge $(L_0)$ zwischen Angriffspunkt (AP) und Drehpunkt (DP) des bettpflichtigen Patienten (90), sowie ein Winkel $(\theta\_DP)$ zwischen zwei Punkten auf der Trajektorie $(T_n; T_{n+1})$ und dem Drehpunkt (DP) ermittelt werden ; wobei

- als Modelfunktion die Koordinatengleichung

$$\frac{(Y_n- M_Y(Y_H))^2}{L_0{}^2} + \frac{(Z_n- M_Z(Z_H))^2}{(L_0 \pm \Delta L)^2} = 1 \quad \text{für eine Ellipse;}$$

- als Parameter für den Mittelpunkt der Ellipse eine Funktion $(M_Y(Y_H); M_Z(Z_H))$ in Abhängigkeit von der Position $(Y_H(t) / Z_H(t))$ des Drehpunkts (DP), insbesondere des Hüftgelenks (98); und

- als große Halbachse der Ellipse die Länge $(L_0)$ zwischen Angriffspunkt (AP) und Drehpunkt (DP) des bettpflichtigen Patienten (90), wobei der Term $(L_0 \pm \Delta L)$ eine mögliche Verschiebung der Position $(Y_H(t)/Z_H(t))$ des Drehpunkts (DP) während einer Bewegung beschreibt,

zugrunde gelegt wird.

Dabei kann der Angriffspunkt auch vorteilhaft das Kniegelenk des Patienten sein.

Die Bestimmung der Trajektorie allein des Angriffspunkts, insbesondere des Kniegelenks, während einer Bewegung, insbesondere während einer ersten, durch einen Therapeuten geführten Bewegung oder einer automatisiert durchgeführten Bewegung, erlaubt vorteilhaft die Erstellung eines kinematischen Modells der Längenverhältnisse, der Position und/oder des Bewegungsradius wenigstens von Teilen der unteren Extremitäten des Patienten aus den dabei bestimmten Daten. Das Kniemodul eines Rehabilitationsmechanismus kann diese Trajektorie anschließend vorteilhaft selbstständig abfahren und dabei die unteren Extremitäten, insbesondere das Bein, eines bettpflichtigen Patienten führen. Somit ist eine an die anatomischen Verhältnisse des jeweiligen Patienten angepasste Gangtherapie des Patienten unabhängig von der Verfügbarkeit von therapeutischem Personal möglich.

Das bei der ersten Bewegung erzeugte kinematische Modell liefert zudem auch vorteilhaft anatomische Parameter des Patienten, wie bspw. die Länge des Oberschenkels, die Position des Hüftgelenks, sowie den aktuellen Bewegungsradius des Patienten. Diese Parameter können nicht nur in anderen Therapieformen, zur Steuerung anderer, insbesondere automatisch betriebener Therapiegeräte (Roboter), verwendet werden, sie erlauben auch bei regelmäßiger, zeitlich beabstandeter Messung den Behandlungsverlauf zu verfolgen und die jeweilige Therapiebewegung anzupassen.

Die Kurvenanpassung, insbesondere basierend auf einer Least-Squares-Optimierung der Messdaten ausgehend von einer Modellfunktion oder auch einer sog. Spline-Interpolation, an die jeweils bestimmte Trajektorie des Angriffspunkts, insbesondere des Kniegelenks, ermöglicht vorteilhaft die Bestimmung der Position für den Drehpunkt, insbesondere für das Hüftgelenk, sowie die Länge zwischen Angriffspunkt (AP) und Drehpunkt (DP), insbesondere die Länge des Oberschenkels, lediglich aus der zuvor aufgezeichneten Trajektorie des Angriffspunkts, insbesondere des Kniegelenks, während der manuell und/oder automatisiert geführten Bewegung. Diese "initialisierende" Bewegung ist obligatorisch und müsste vom Therapeuten in jedem Fall zur Bestimmung des aktuell vom Patienten bewältigbaren Bewegungsradius ausgeführt werden.

Dadurch, dass als Modellfunktion dazu die Koordinatengleichung $\frac{(Y_n - M_Y(Y_H))^2}{L_0{}^2} + \frac{(Z_n - M_Z(Z_H))^2}{(L_0 \pm \Delta L)^2} = 1$ für eine Ellipse; als Parameter für den Mittelpunkt der Ellipse eine Funktion in Abhängigkeit von der Position des Drehpunkts und als große Halbachse der Ellipse die Länge zwischen Angriffspunkt und Drehpunkt Länge des Oberschenkels des bettpflichtigen Patienten zugrunde gelegt werden, kann vorteilhaft einer "variablen" sich während einer Bewegung veränderlichen Position des Drehpunkts, insbesondere des Hüftgelenks, Rechnung getragen werden - eine Situation, wie sie insbesondere bei älteren Patienten vorkommen kann.

Bei (jungen) Patienten, mit einer näherungsweise ortsfesten Position des Drehpunkts, insbesondere des Hüftgelenks, kann sich die Modellfunktion zu einer Koordinatengleichung $(Y_n - Y_H)^2 + (Z_n - Z_H)^2 = L_0{}^2$ für einen Kreis vereinfachen. Als Parameter für den Mittelpunkt des Kreises kann dabei die Position des Drehpunkts, insbesondere des Hüftgelenks; und als Radius des Kreises die Länge zwischen Angriffspunkt und Drehpunkt, insbesondere die Länge des Oberschenkels, des bettpflichtigen Patienten zugrunde gelegt werden.

Die Verwendung der Koordinatengleichung eines Kreises als Modellfunktion kann vorteilhaft eine rechnerisch einfache Möglichkeit zur Bestimmung der Position des Drehpunkts, insbesondere des Hüftgelenks, sowie der Länge zwischen Angriffspunkt und Drehpunkt, insbesondere der Länge des Oberschenkels des Patienten, bieten, wohingegen die Verwendung anderer, komplexerer Modellfunktion wie beispielsweise die Koordinatengleichung einer Ellipse Fälle vorteilhaft abbilden kann, bei denen sich die Position des Drehpunkts, insbesondere des Hüftgelenks des Patienten während der Bewegung des Beins leicht verändert (variiert).

Bevorzugt wird dabei in einem Verfahrensschritt das Bein über einen Angriffspunkt, insbesondere über das Kniegelenk, den Oberschenkel und/oder einen Unterschenkel mittels einer Knie-Orthese am Kniemodul fixiert. Die Fixierung des Beins am Kniemodul ermöglicht vorteilhaft zunächst, während des Mess- und/oder Bestimmungsvorgangs der Trajektorie des Kniegelenks, die Kraftübertragung vom Bein auf das Kniemodul und somit auf die Messeinrichtung während der ersten, geführten Bewegung des Beins und anschließend, während der Therapie, die Kraftübertragung vom Kniemodul auf das Bein während der automatischen Ausführung der Therapiebewegung.

Darüber hinaus ist erfindungsgemäß bevorzugt, dass in einem Verfahrensschritt die Messeinrichtung ausgehend von einer Ausgangposition eine manuell und/oder automatisiert geführte Bewegung des Beins, insbesondere als Trajektorie eines Angriffspunkts, insbesondere des Kniegelenks, in Form von Koordinatenpaaren oder zeitabhängig in Form von Koordinatentripeln, aufzeichnet. Die Festlegung einer Ausgangsposition erfolgt manuell durch den Therapeuten in Abhängigkeit der Bewegungsmöglichkeiten des jeweiligen Patienten oder aber automatisiert anhand plausibler, geschätzter Werte der anatomischen Verhältnisse des jeweiligen Patienten. Die sich dabei ergebende Position des Angriffspunkts, insbesondere des Kniegelenks, kann im weiteren Ablauf vorteilhaft den Bezugspunkt für weitere Bewegungen bilden.

Dabei hat sich in einer Ausgestaltung bewährt, dass die Messeinrichtung die Trajektorie des Angriffspunkts, insbesondere des Kniegelenks, in Form von Koordinatenpaaren oder zeitabhängig in Form von Koordinatentripeln mit Hilfe von wenigstens zwei Sensoren aus den Winkelpositionen von elektromotorisch angetriebenen Exzentern bestimmt. Durch Bestimmung der Koordinatenpaare bzw. der Koordinatentripel der Trajektorie des Angriffspunkts, insbesondere des Kniegelenks, aus den Winkelpositionen der Exzenter können vorteilhaft andere Sensoren eingespart werden. Dies kann vorteilhaft die Kosten für zusätzliche Sensortechnik, sowie den Zeitaufwand für eine sensorbasierte und/oder manuelle Messung einsparen und gleichzeitig vergleichsweise genaue, reproduzierbare Messergebnisse liefern, welche wiederum zur Generierung eines möglichst genauen, reproduzierbaren Modells des bettpflichtigen Patienten Verwendung finden können.

In einer weiteren Ausgestaltung der Erfindung hat sich bewährt, wenn in einem Verfahrensschritt ein Steuermodul zur Aussteuerung planmäßiger Rehabilitationsbewegungen zumindest der Gelenke, Muskeln und Sehnen der Beine des bettpflichtigen Patienten mittels des Kniemoduls vorgesehen ist, wobei das Steuermodul vorzugsweise aus den, aus dem kinematischen Modell generierten, patientenspezifischen Daten, Trajektorien, insbesondere neue, veränderte Trajektorien, für die Rehabilitationsbewegungen des Beines eines Patienten bezüglich des Angriffspunkts, insbesondere des Kniegelenks bestimmt und anhand dieser Trajektorien das Kniemodul, vorzugsweise in Abhängigkeit von Nutzereingaben, aussteuert. Nach der einmaligen Aufzeichnung der manuell und/oder automatisiert geführten Trajektorie des Angriffspunkts, insbesondere des Kniegelenks, kann dann während der eigentlichen Bewegungstherapie die zuvor aufgezeichnete Trajektorie reproduziert und/oder entsprechend der neuen, veränderten Trajektorien veränderte Bewegungen mit Hilfe des Rehabilitationsmechanismus und/oder Kniemoduls ausgeführt werden. Die Bewegungstherapie des bettpflichtigen Patienten kann dadurch unabhängig von der zeitlichen Verfügbarkeit von Pflegepersonal, jederzeit und beliebig oft durchgeführt werden.

Von Vorteil ist zudem eine Ausgestaltung, wenn der Rehabilitationsmechanismus und/oder das Kniemodul während der manuell und/oder automatisiert geführten Bewegung des Beins mit Hilfe des Steuermoduls derart gesteuert wird, dass die Einwirkung von Kräften entlang einer durch Angriffspunkt und Drehpunkt definierten Achse durch den Rehabilitationsmechanismus und/oder das Kniemodul auf das Bein des bettpflichtigen Patienten vermieden ist. Dadurch kann vorteilhaft bei der erstmaligen, manuell vom Therapeuten und/oder automatisiert geführten Bewegung eine Verfälschung der Messwerte durch einen Einfluss des Rehabilitationsmechanismus und/oder durch das Kniemodul auf die natürliche Bewegung des Patienten vermieden werden. Darüber hinaus kann eine derartige Steuerung die Bedienung des Rehabilitationsmechanismus und/oder des Kniemoduls erleichtern, da mechanische Widerstände des Apparats vom Therapeuten nicht überwunden werden müssen.

Darüber hinaus hat sich eine Ausgestaltung des erfindungsgemäßen Messverfahrens bewährt, bei der das Steuermodul aufgrund berechneter Trajektorien für den Angriffspunkt, insbesondere für das Kniegelenk, das Kniemodul und/oder den Rehabilitationsmechanismus derart steuert, dass der Angriffspunkt, insbesondere das Kniegelenk, des bettpflichtigen Patienten mit Hilfe des Kniemoduls und ggf. der Knie-Orthese entlang dieser, insbesondere neuen, veränderten, Trajektorien bewegt wird. Ohne neue Messungen vornehmen zu müssen kann so der Bewegungsablauf während der physiotherapeutischen Übungen automatisch an den Therapieverlauf, insbesondere bezüglich eines sich verändernden Bewegungsradius bzw. Bewegungsraumes des Patienten, angepasst werden.

In einer erfindungsgemäß bevorzugten Ausgestaltung hat sich zudem bewährt, wenn in einem Verfahrensschritt nach jeder automatisiert geführten Bewegung des Beins, aus jeder neuen, zeitabhängig aufgezeichneten Trajektorie des Angriffspunkts neue Werte für die Länge zwischen Angriffspunkt und Drehpunkt, die Position des Drehpunkts, sowie den Winkel zwischen zwei Punkten auf der Trajektorie und dem Drehpunkt berechnet und mit den korrespondierenden Werten zuvor gemessener Trajektorien verglichen werden.

Erfindungsgemäß bevorzugt ist darüber hinaus, wenn in einem Verfahrensschritt, anhand des Vergleichs der Werte für die Länge zwischen Angriffspunkt und Drehpunkt, die Position des Drehpunkts, sowie den Winkel zwischen zwei Punkten auf der Trajektorie und dem Drehpunkt von je zwei, zeitlich aufeinander folgend gemessenen Trajektorien mittels eines Optimierungs-Algorithmus, insbesondere eines *Least-Squares*-Algorithmus, ein aktualisiertes Modell mit verbesserten Werten für die Länge zwischen Angriffspunkt und Drehpunkt, die Position des Drehpunkts, sowie einen Winkel zwischen zwei Punkten auf der Trajektorie und dem Drehpunkt erzeugt wird.

Schließlich hat sich eine Ausgestaltung bewährt, wenn in einem Verfahrensschritt die Werte des aktualisierten Modells als Basis für eine weitere, automatisiert geführte Bewegung des Beines dienen.

Werden nach jeder automatisiert geführten Bewegung des Beins, aus jeder neuen, zeitabhängig aufgezeichneten Trajektorie des Angriffspunkts neue Werte für die Länge zwischen Angriffspunkt und Drehpunkt, die Position des Drehpunkts, sowie der Winkel zwischen zwei Punkten auf der Trajektorie und dem Drehpunkt berechnet, mit den gemessenen Werten vorheriger Zyklen verglichen und unter Berücksichtigung der Trajektorien vorheriger Zyklen, also vorheriger automatisiert durchgeführter Bewegungen des Beins eines Patienten, optimiert und die so optimierten Werte für die Länge zwischen Angriffspunkt und Drehpunkt, die Position des Drehpunkts, sowie der Winkel zwischen zwei Punkten auf der Trajektorie und dem Drehpunkt als Basis für eine weitere, automatisiert geführte Bewegung des Beins genutzt, kann die durch den Rehabilitationsmechanismus automatisiert geführte Therapiebewegung während der Durchführung an die anatomischen Verhältnisse des jeweiligen Patienten vorteilhaft angepasst werden. Der Rehabilitationsmechanismus hat dadurch zudem die Möglichkeit auf Abweichungen, beispielsweise aufgrund eines Verrutschens der Knieorthese und/oder einer Bewegung des gesamten Körpers des Patienten bezüglich des Rehabilitationsmechanismus, oder anderweitigen Veränderungen zeitnah, insbesondere nach jedem Zyklus, also nach einer automatisiert durchgeführter vollständig Therapiebewegungen des Beins eines Patienten, ebenso automatisiert zu reagieren und - gegebenenfalls anhand von Fehlergrenzwerten - Korrekturen an der automatisiert geführten Therapiebewegung vorzunehmen.

Diese Möglichkeit des "machine learning" ermöglicht es vorteilhaft dem Patienten mit Hilfe eines Rehabilitationsmechanismus eine individualisierte Bewegungstherapie zu bieten, welche aufgrund der vorgesehenen Fehlerkorrekturmöglichkeit robust gegenüber äußeren Störungen ist, seien sie verursacht durch Veränderungen am Gerät selbst oder aber durch Veränderungen des Patienten während der Benutzung.

Eine erfindungsgemäße Vorrichtung zur Durchführung eines solchen Messverfahrens zeichnet sich gegenüber gattungsbildenden Vorrichtungen durch die Merkmale des Anspruchs 13 aus.

Die erfindungsgemäße Vorrichtung ermöglicht vorteilhaft die automatische Aufzeichnung und/oder der Bestimmung einer Trajektorie des Angriffspunktes, insbesondere des Kniegelenks, während der Bewegung des Beins eines bettpflichtigen Patienten ausschließlich aus kinematischen Bewegungsdaten und ohne die Verwendung anderer, beispielsweise optischer, Sensoren. Die erfindungsgemäße Vorrichtung ermöglicht zudem vorteilhaft aus den so gewonnenen Daten ein kinematisches Modell der Längenverhältnisse, der Position und/oder des Bewegungsradius wenigstens von Teilen der unteren Extremitäten des Patienten zu erstellen.

In einer bevorzugten Ausgestaltung der Vorrichtung umfasst die Messeinrichtung dabei wenigstens zwei Sensoren zur Bestimmung der Winkelposition der Welle des Elektromotors und/oder der Exzenter. Dadurch wird vorteilhaft keine weitere Sensortechnik benötigt, da insbesondere die Drehgeber von Servomotoren als Sensoren genutzt werden können.

Von Vorteil ist zudem, wenn ein Steuermodul zur Aussteuerung planmäßiger Rehabilitationsbewegungen zumindest der Gelenke, Muskeln und Sehnen der Beine des bettpflichtigen Patienten mittels des Kniemoduls vorgesehen ist, wobei das Steuermodul vorzugsweise eingerichtet ist, aus den, aus dem kinematischen Modell generierten, patientenspezifischen Daten, Trajektorien, insbesondere neue, veränderte Trajektorien, für die Rehabilitationsbewegungen des Beines eines Patienten bezüglich des Angriffspunkts, insbesondere des Kniegelenks, zu bestimmen und anhand dieser Trajektorien das Kniemodul, vorzugsweise in Abhängigkeit von Nutzereingaben, auszusteuern. Mit Hilfe des Steuermoduls kann der Rehabilitationsmechanismus und/oder das Kniemodul vorteilhaft derart ausgesteuert werden, dass die Rehabilitationsbewegung einer zuvor bestimmten Trajektorie folgt.

In einer weiteren bevorzugten Ausgestaltung der Vorrichtung umfasst das Kniemodul wenigstens eine das Kniegelenk des bettpflichtigen Patienten aufnehmende Knie-Orthese; ein mit der Knie-Orthese verbundenes Verbindungselement; einen Ausleger, an welchem das Verbindungselement befestigt ist; und eine mittels eines Steuermoduls ansteuerbare Mechanik; welche über den Ausleger und das Verbindungselement eine definierte Kraft dergestalt in die Knie-Orthese einleitet, dass über den Angriffspunkt, insbesondere das darin aufgenommene Kniegelenk des bettpflichtigen Patienten, die Gelenke, Muskeln und Sehnen dieses Beines planmäßige Rehabilitationsbewegungen ausführen. Eine derartige Ausführung kann vorteilhaft die Bewegung der Knie-Orthese gegen über dem Kniemodul ermöglichen und so die Bewegungsfreiheitsgrade der Therapiebewegung vorteilhaft erhöhen.

Schließlich ist es gemäß einer Ausgestaltung von Vorteil, wenn der Rehabilitationsmechanismus wenigstens einen Winkelsensor, welcher den von einem Verbindungselement zur Knie-Orthese und/oder zu einem Ausleger eingenommenen Winkel überwacht; und/oder einen Kraftsensor, welcher die über den Ausleger und das Verbindungselement in die Knie-Orthese eingeleitete Kraft überwacht, umfasst. Im Fall einer im Bezug zum Kniemodul beweglichen Knie-Orthese, was vorteilhaft die Zahl der Bewegungsfreiheitsgrade für die Therapiebewegung erhöhen kann, ermöglicht der Winkelsensor in Kombination mit den Sensoren die automatische Aufzeichnung und/oder Bestimmung der Trajektorie des Kniegelenks.

Die vorliegende Erfindung ermöglicht vorteilhaft die Bestimmung der Längenverhältnisse, der Position und/oder des Bewegungsradius der unteren Extremitäten eines bettpflichtigen Patienten, wie Hüftgelenk, Kniegelenk und/oder Oberschenkel ohne operativen Eingriff allein anhand der Aufzeichnung und/oder Bestimmung der Trajektorie eines Angriffspunktes, insbesondere des Kniegelenks, während wenigstens einer initialisierenden Bewegung des Patienten. Unter Einsparung aufwendiger und teurer Sensortechnik stellt die Erfindung vorteilhaft zudem ein kinematisches Modell des Bewegungsradius des Patienten bereit, dessen Parameter als Grundlage zur Erstellung neuer Therapieabläufe innerhalb seiner Bewegungstherapie, sowie als Ausgangsmodell für andere automatisierte Therapie- und Untersuchungsmethoden dienen können. Die vorliegende Erfindung erlaubt darüber hinaus über eine "machine learning"-Funktionalität dem Patienten mit Hilfe eines Rehabilitationsmechanismus eine individualisierte Bewegungstherapie zu bieten, welche aufgrund der vorgesehenen Fehlerkorrekturmöglichkeit robust gegenüber äußeren Störungen ist.

Zusätzliche Einzelheiten und weitere Vorteile werden nachfolgend an Hand bevorzugter Ausführungsbeispiele, auf welche die vorliegende Erfindung jedoch nicht beschränkt ist, und in Verbindung mit der beigefügten Zeichnung beschrieben.

Darin zeigen schematisch:

Fig. 1     ein erstes Verlaufsdiagramm eines Messverfahrens zur Bestimmung der Längenverhältnisse, der Position und/oder des Bewegungsradius der unteren Extremitäten eines bettpflichtigen Patienten, wie Hüftgelenk, Kniegelenk und/oder Oberschenkel;

Fig. 2a     einen Torso sowie ein Bein eines bettpflichtigen Patienten bei einer möglichen Bewegung des Beines während der Bewegungstherapie;

Fig. 2b     eine sich aus der Bewegung aus Fig. 2a ergebenden Trajektorie des Kniegelenks anhand dreier Koordinatenpaare innerhalb eines zugrunde gelegten Koordinatensystems, sowie eine mögliche Modellfunktion vor der Kurvenanpassung;

Fig. 2c     die Trajektorie des Kniegelenks aus Fig. 2b mit angepasster Modellfunktion, hier in Form eines Kreises, sowie den aus der Anpassung generierten Parametern für die Länge eines Oberschenkels und für die Position des Hüftgelenks;

Fig. 3     die Anpassung der Modellfunktion einer Ellipse an die Koordinatenpaare einer Trajektorie des Kniegelenks;

Fig. 4      einen linken Ausleger eines Kniemodul eines Rehabilitationsmechanismus einer Vorrichtung zur Durchführung des erfindungsgemäßen Verfahrens in einer perspektivischen Ansicht;

Fig. 5      beispielhaft den Ausleger aus Fig. 4 in einer vergrößerten Seitenansicht;

Fig. 6      einen gesicherten, bettpflichtigen Patienten in einem handelsüblichen Bett nach dem Stand der Technik, insb. Intensivbett, mit angelegtem Knie- und Fußmodul eines Rehabilitationsmechanismus vor einer Vertikalisierung des Bettes in einer perspektivischen Seitenansicht;

Fig. 7a bis c      eine von dem Rehabilitationsmechanismus über die Knie-Orthese erzeugte Trajektorie zum Beugen bzw. Strecken der Beine eines Patienten;

Fig. 8      eine Trajektorie eines Angriffspunktes an einem Oberschenkel eines Patienten mit angepasster Modellfunktion und die bei einem Anheben eines Beines durch einen Rehabilitationsmechanismus und/oder ein Kniemodul über den Angriffspunkt auf das Bein des Patienten ausgeübte Kraft, sowie die Einwirkung von Kräften entlang einer durch Angriffspunkt und Drehpunkt definierten Achse auf das Bein des bettpflichtigen Patienten;

Fig. 9      im Rahmen einer Kurvenanpassung ("curve-fitting") ermittelte Winkel zwischen jeweils zwei Punkten auf der Trajektorie und dem Drehpunkt, hier speziell einen Winkel $\theta_{max}$ bei maximaler Anhebung des Angriffspunktes und einen Winkel $\theta_{min}$ bei minimaler Anhebung des Angriffspunktes; und

Fig. 10 a und b      ein zweites Verlaufsdiagramm (aufgeteilt auf die beiden Figuren) eines erfindungsgemäß bevorzugten Messverfahrens zur Bestimmung der Längenverhältnisse, der Position und/oder des Bewegungsradius der unteren Extremitäten eines bettpflichtigen Patienten, wie Hüftgelenk, Kniegelenk und/oder Oberschenkel mit der Möglichkeit einer automatischen Fehlerkorrektur und schnelle Anpassung an äußere Störungen.

Bei der nachfolgenden Beschreibung bevorzugter Ausführungsformen bezeichnen gleiche Bezugszeichen gleiche oder vergleichbare Komponenten.

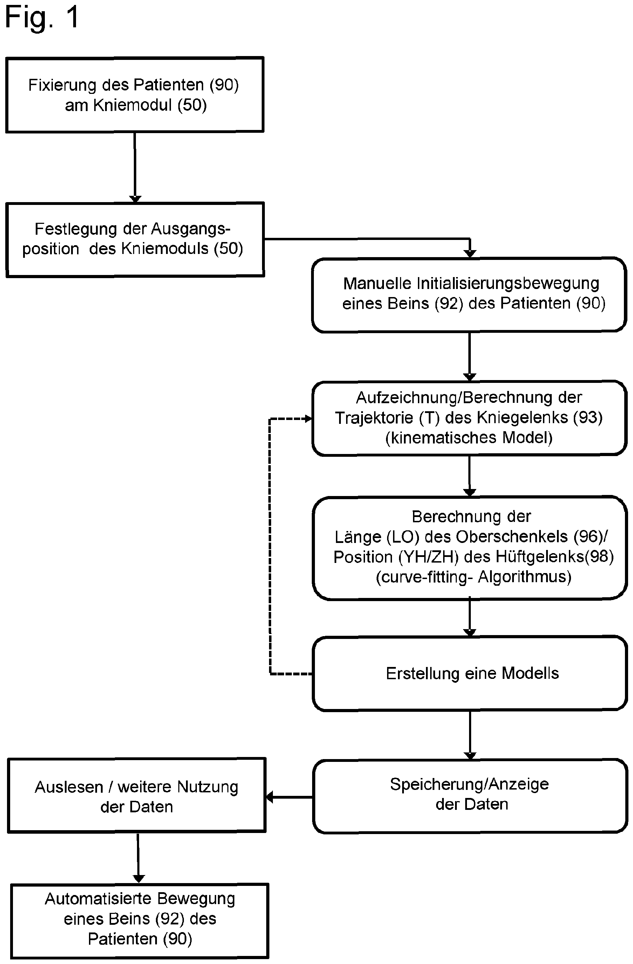

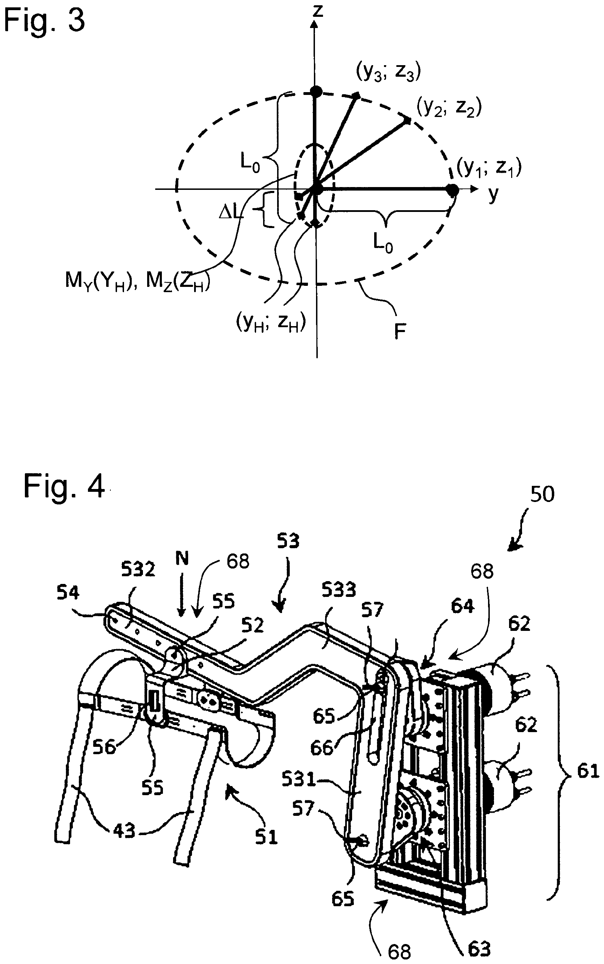

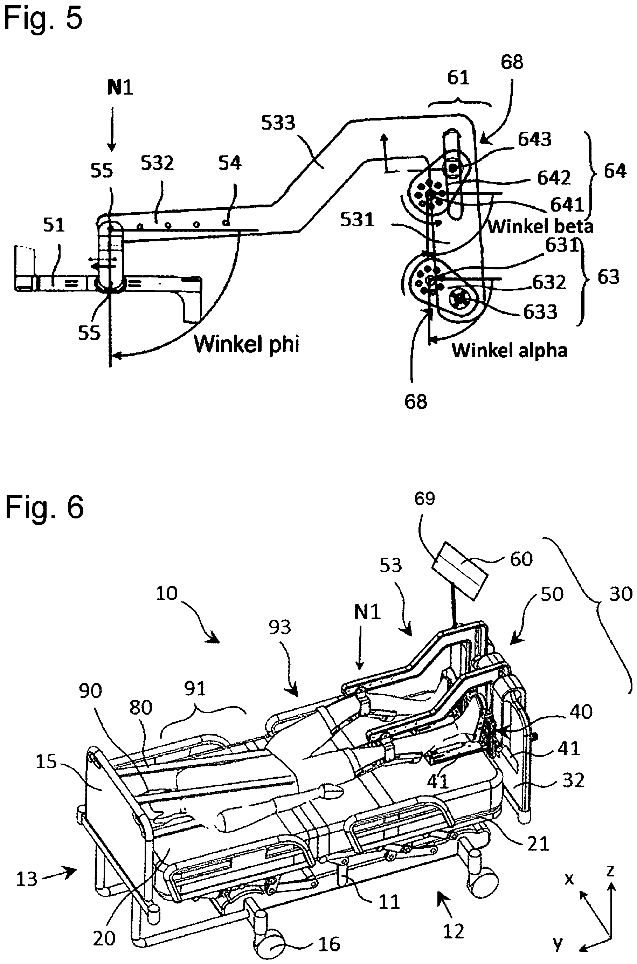

**Fig. 1** zeigt ein Verlaufsdiagramm eines Messverfahrens zur Bestimmung der Längenverhältnisse, der Position und/oder des Bewegungsradius der unteren Extremitäten eines bettpflichtigen Patienten 90, wie Hüftgelenk 98, Kniegelenk 93 und/oder Oberschenkel 96. Zunächst wird wenigstens ein Kniemodul 50 eines Rehabilitationsmechanismus 30 mit einem Kniegelenk 93 des Patienten 90 in Wirkverbindung gebracht. Dazu kann das Bein 92, insbesondere das Kniegelenk 93, der Oberschenkel 96 und/oder ein Unterschenkel 97 beispielsweise mittels einer Knie-Orthese 51 am Kniemodul 50 fixiert werden (vgl. auch Fig. 4 und Fig. 6).

In einem zweiten Verfahrensschritt kann dann mittels einer Messeinrichtung 68, eine Trajektorie T des Kniegelenks 93 während einer Bewegung des Beines 92, insbesondere während einer von einem Therapeuten durchgeführte Initialisierungsbewegung, mit dem Kniemodul 50 des Rehabilitationsmechanismus 30 aufgezeichnet und/oder bestimmt werden. Während dieser Initialisierungsbewegung kann es von Vorteil sein, wenn der Rehabilitationsmechanismus 30 und/oder das Kniemodul 50 während der manuell geführten Bewegung des Beins 92 mit Hilfe des Steuermoduls 60 derart gesteuert werden, dass die Einwirkung von Kräften N durch den Rehabilitationsmechanismus 30 und/oder das Kniemodul 50 auf das Bein 92 des bettpflichtigen Patienten 90 vermieden ist.

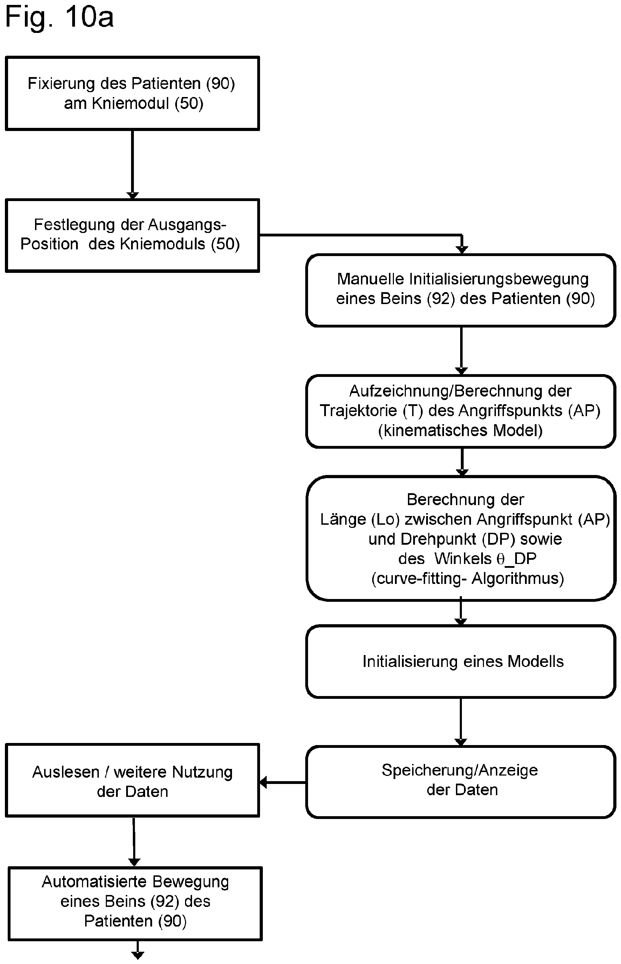

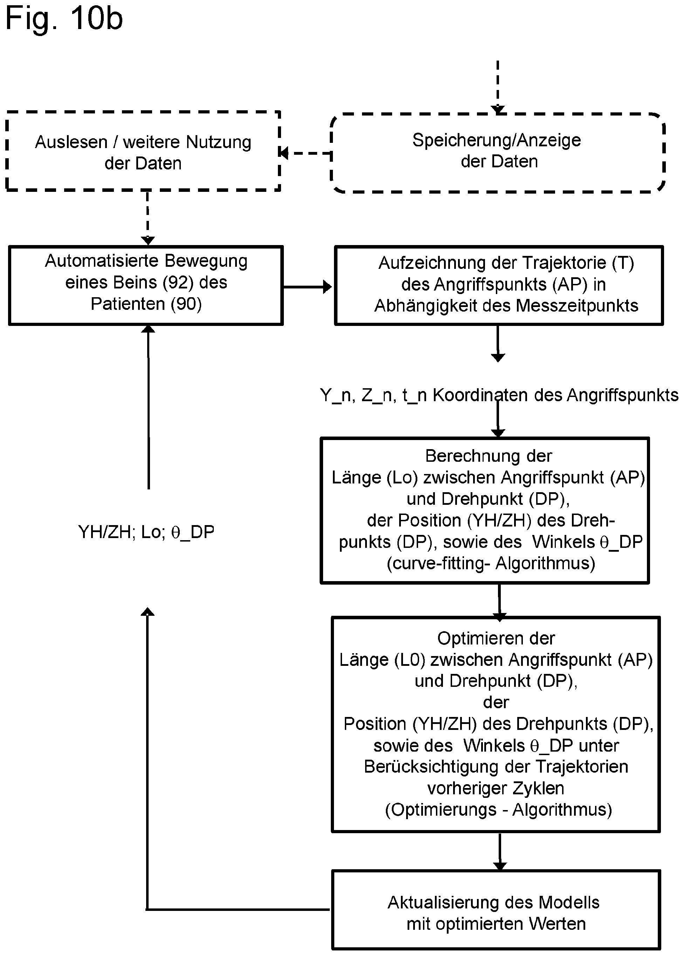

In den **Fig. 10a** und **10b** ist ein zweites Verlaufsdiagramm (aufgeteilt auf die beiden Figuren) eines erfindungsgemäß bevorzugten Messverfahrens zur Bestimmung der Längenverhältnisse, der Position und/oder des Bewegungsradius der unteren Extremitäten eines bettpflichtigen Patienten 90, wie Hüftgelenk 98, Kniegelenk 93 und/oder Oberschenkel 96 mit der Möglichkeit einer automatischen Fehlerkorrektur und schnelle Anpassung an äußere Störungen gezeigt. Die Initialisierung eines Modells erfolgt auf eine mit der im ersten Verlaufsdiagramm der Fig.1 dargestellten vergleichbaren Weise, wobei die Messeinrichtung 68 diesmal ausgehend von einer Ausgangposition Po eine manuell und/oder automatisiert geführte Bewegung des Beins 92, insbesondere als Trajektorie $T_n$; $T_{n+1}$ eines Angriffspunkts AP in Form von Koordinatentripeln $Y_1/ Z_1/ t_1$, $Y_2/ Z_2/ t_2$, ..., $Y_n/ Z_n/ t_n$ zeitabhängig aufzeichnet. Als Angriffspunkt AP, über den wenigstens ein Kniemodul 50 eines Rehabilitationsmechanismus 30 mit einem Bein 92 des Patienten 90 in Wirkverbindung gebracht wird, kann vorzugsweise das Kniegelenk 93, der Ober- 96 oder Unterschenkel 97, oder aber irgendein Punkt des Beins 92 gewählt werden.

Die Messeinrichtung 68 kann die Trajektorie $T_n$; $T_{n+1}$ des Angriffspunkts AP, insbesondere des Kniegelenks 93, wiederum in Form von Koordinatentripeln $Y_1/ Z_1/ t_1$, $Y_2/ Z_2/ t_2$, ..., $Y_n/ Z_n/ t_n$ mit Hilfe von wenigstens zwei Sensoren 681; 682 aus den Winkelpositionen von elektromotorisch angetriebenen Exzentern 63; 64 bestimmen.

Die zeitabhängige Aufzeichnung der Trajektorie $T_n$; $T_{n+1}$ eines Angriffspunkts AP in Form von Koordinatentripeln $Y_1/ Z_1/ t_1$, $Y_2/ Z_2/ t_2$, ..., $Y_n/ Z_n/ t_n$ bietet dabei erstens den Vorteil, dass durch Zuordnung eines Messzeitpunktes $t_1$, $t_2$, ..., $t_n$ zu den gemessenen Koordinatenpaaren $Y_1/Z_1$, ..., $Y_n/Z_n$ vorteilhaft zwischen einer Hebe- und einer Senkbewegung des Beines 92 unterschieden werden kann. Zweitens kann die auf diese Weise bestimmbare Reihenfolge der Messwerte während der Optimierung mittels eines Optimierungs-Algorithmus vorteilhaft berücksichtigt werden und so die Optimierungsergebnisse verbessern. Dem liegt die Überlegung zugrunde, dass zeitlich nahe beieinanderliegende Messwerte auch auf einer gemessenen Trajektorie $T_n$; $T_{n+1}$ eher benachbart sein sollten und somit auch ähnliche physikalische Eigenschaften aufweisen sollten.

Im Anschluss an die Initialisierung können nun nach jeder automatisiert geführten Bewegung des Beins 92, aus jeder neuen, zeitabhängig aufgezeichneten Trajektorie $T_{n+1}$ des Angriffspunkts AP neue Werte für die Länge Lo zwischen Angriffspunkt AP und Drehpunkt DP, die Position $Y_H(t) / Z_H(t)$ des Drehpunkts DP, sowie den Winkel θ_DP zwischen zwei Punkten auf der Trajektorie $T_{n+1}$ und dem Drehpunkt DP berechnet und mit den korrespondierenden Werten zuvor gemessener Trajektorien $T_n$ verglichen werden. Die Position $Y_H(t) / Z_H(t)$ des Drehpunkts DP, wobei es sich beim Drehpunkt DP beispielsweise um das Hüftgelenk 98 des Patienten 90 handeln kann, kann dabei eine näherungsweise ortsfeste Position $Y_H(t) / Z_H(t)$ mit $Y_H(t_1) = Y_H(t_2) =...= Y_H(t_n)$ und $Z_H(t_1) = Z_H(t_2) =... = Z_H(tn)$ im Raum, also ein wirklicher Dreh-"Punkt", sein, oder aber, wie es öfters insbesondere bei älteren Patienten 90 aufgrund einer Abnutzung der Hüftgelenkpfanne der Fall sein kann, um eine "variable", sich während einer Bewegung verändernden, zeitabhängige Position $Y_H(t) / Z_H(t)$ des Drehpunkts DP, insbesondere des Hüftgelenks 98 des Patienten 90, sein. Anhand des Vergleichs der Werte für die Länge Lo zwischen Angriffspunkt AP und Drehpunkt DP, die Position YH(t) / ZH(t) des Drehpunkts DP, sowie den Winkel zwischen zwei Punkten auf der Trajektorie $T_n$; $T_{n+1}$ und dem Drehpunkt DP von je zwei, zeitlich aufeinander folgend gemessenen Trajektorien $T_n$, $T_{n+1}$ mittels eines Optimierungs-Algorithmus, insbesondere eines *Least-Squares*-Algorithmus, kann dann ein aktualisiertes Modell mit verbesserten Werten für die Länge Lo zwischen Angriffspunkt AP und Drehpunkt D, die Position YH(t) / ZH(t) des Drehpunkts DP, sowie einen Winkel θ_DP zwischen zwei Punkten auf der Trajektorie $T_n$; $T_{n+1}$ und dem Drehpunkt DP erzeugt werden, dessen Werte vorteilhaft als Basis für eine weitere, automatisiert geführte Bewegung des Beines 92 dienen können.

Das Messverfahren ermöglicht so einer Vorrichtung 1 zur Durchführung eines solchen Messverfahrens vorteilhaft das maschinelle Lernen ("machine learning") einer Therapiebewegung während des automatisierten Bewegungsablaufs ("Zyklus"). Diese Möglichkeit des "machine learning" ermöglicht es vorteilhaft dem Patienten 90 mit Hilfe eines Rehabilitationsmechanismus 30 eine individualisierte Bewegungstherapie zu bieten, welche aufgrund der vorgesehenen Fehlerkorrekturmöglichkeit robust gegenüber äußeren Störungen ist, seien sie verursacht durch Veränderungen am Rehabilitationsmechanismus 30 selbst oder aber durch Veränderungen des Patienten 90 während der Benutzung.

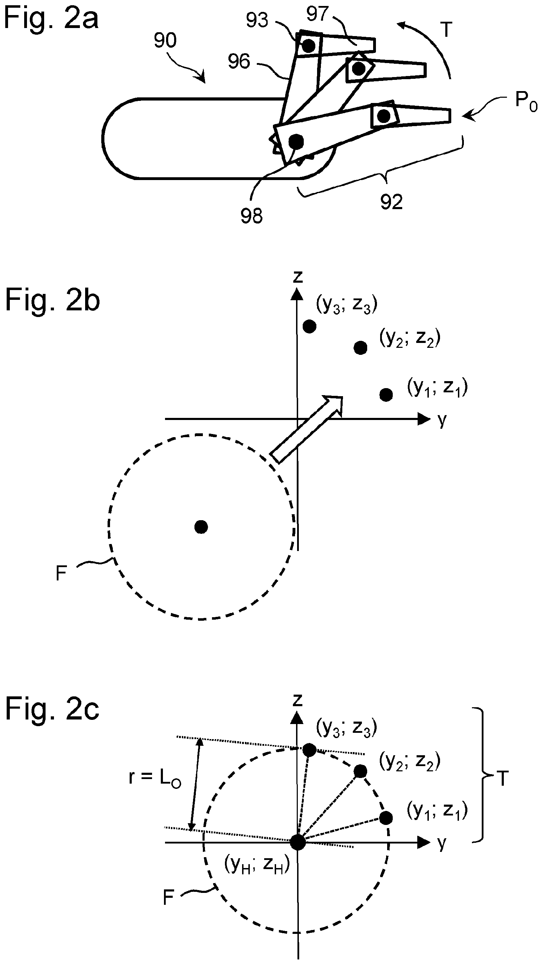

Die Fig. 2a bis 2c verdeutlichen diesen Aufzeichnungs- und/oder Bestimmungsvorgang.

**Fig. 2a** zeigt einen Torso sowie ein Bein 92 eines bettpflichtigen Patienten 90 bei einer möglichen Bewegung des Beines 92 während der Bewegungstherapie.

In **Fig. 2b** ist eine sich aus dieser Bewegung ergebende Trajektorie T des Kniegelenks 93 anhand dreier Koordinatenpaare $Y_n/Z_n$ innerhalb eines zugrunde gelegten Koordinatensystems, sowie eine mögliche Modellfunktion F vor der Kurvenanpassung dargestellt. Die Trajektorie T des Kniegelenks 93 kann vorzugsweise von einer Messeinrichtung 68 ausgehend von einer Ausgangposition Po während einer manuell geführten Bewegung des Beins 92, insbesondere in Form von Koordinatenpaaren $Y_1/ Z_1$, $Y_2/ Z_2$, ..., $Y_n/ Z_n$, aufgezeichnet werden. Die Bestimmung der Koordinatenpaare $Y_1/ Z_1$, $Y_2/ Z_2$, ..., $Y_n/ Z_n$ kann dabei vorteilhaft mit Hilfe von wenigstens zwei Sensoren 681; 682 aus den Winkelpositionen von elektromotorisch angetriebenen Exzentern 63; 64 erfolgen (vgl. Fig. 4, 5, 7a bis c).

Die Bestimmung der Trajektorie T erfolgt dabei vorteilhaft durch einen Vergleich der ermittelten Koordinatenpaare $Y_1/ Z_1$; $Y_2/ Z_2$; .../...; $Y_n/ Z_n$ mit einer Modelfunktion in Form einer Koordinatengleichung.

Als Modellfunktion F kommt beispielsweise eine Koordinatengleichung $(Y_n - Y_H)^2 + (Z_n - Z_H)^2 = L_0^2$ für einen Kreis mit der Position $Y_H/ Z_H$ des Hüftgelenks 98 als Mittelpunkt des Kreises und mit der Länge $L_0$ des Oberschenkels 96 des bettpflichtigen Patienten 90 als dessen Radius in Frage.

**Fig. 2c** zeigt die Trajektorie T des Kniegelenks 93 aus Fig. 2b mit angepasster Modellfunktion F in Form der Koordinatengleichung eines Kreises, sowie den aus der Anpassung generierten Parametern für die Länge $L_0$ eines Oberschenkels 96 und für die Position $Y_H/ Z_H$ des Hüftgelenks 98.

Alternativ dazu kann als Modellfunktion F auch die Koordinatengleichung
$$\frac{(Y_n - M_Y(Y_H))^2}{L_0^2} + \frac{(Z_n - M_Z(Z_H))^2}{(L_0 \pm \Delta L)^2} = 1$$
für eine Ellipse zugrunde gelegt werden, wobei sich nun als Parameter für den Mittelpunkt der Ellipse eine Funktion $M_Y(Y_H)/M_Z(Z_H)$ in Abhängigkeit von der Position $Y_H/ Z_H$ des Hüftgelenks 98 und als große Halbachse der Ellipse die Länge $L_0$ des Oberschenkels 96 des bettpflichtigen Patienten 90 ergeben kann. Der Term $L_0 \pm \Delta L$ beschreibt eine mögliche Verschiebung der Position $Y_H$/ $Z_H$ des Hüftgelenks 98 während der Bewegung.

In **Fig. 3** ist eine solche Anpassung der Modellfunktion F einer Ellipse an die Koordinatenpaare $Y_n$/ $Z_n$ einer Trajektorie T des Kniegelenks 93 beispielhaft gezeigt, wobei die sich aus der Verschiebung der Position $Y_H$/ $Z_H$ des Hüftgelenks 98 ergebende Funktion $M_Y(Y_H)$/$M_Z(Z_H)$ ebenfalls, wie zu sehen, als eine elliptische Funktion, allerdings mit einer zur großen Halbachse der Modellellipse senkrecht stehenden großen Halbachse, darstellen kann.

Mittels eines Modellgenerierungsmittels 69 kann schließlich aus den so erhaltenen Daten ein kinematisches Modell der Längenverhältnisse, der Position und/oder des Bewegungsradius wenigstens von Teilen der unteren Extremitäten des Patienten 90 erstellt werden.

Mit Hilfe eines Steuermoduls 60 zur Aussteuerung planmäßiger Rehabilitationsbewegungen zumindest der Gelenke, Muskeln und Sehnen der Beine 92 des bettpflichtigen Patienten 90 mittels des Kniemoduls 50 können dann in einem weiteren Verfahrensschritt, vorzugsweise aus den, aus dem kinematischen Modell generierten, patientenspezifischen Daten, Trajektorien T, insbesondere neue, veränderte Trajektorien T, für die Rehabilitationsbewegungen des Kniegelenks 93 bestimmt und anhand dieser Trajektorien T das Kniemodul 50, vorzugsweise in Abhängigkeit von Nutzereingaben, ausgesteuert werden. Das Steuermodul 60 kann dabei aufgrund der berechneten Trajektorien T für das Kniegelenk 93 das Kniemodul 50 und/oder den Rehabilitationsmechanismus 30 derart steuern, dass das Kniegelenk 93 des bettpflichtigen Patienten 90 mit Hilfe des Kniemoduls 50 und ggf. der Knie-Orthese 51 entlang dieser, insbesondere neuen, veränderten, Trajektorien T bewegt wird.

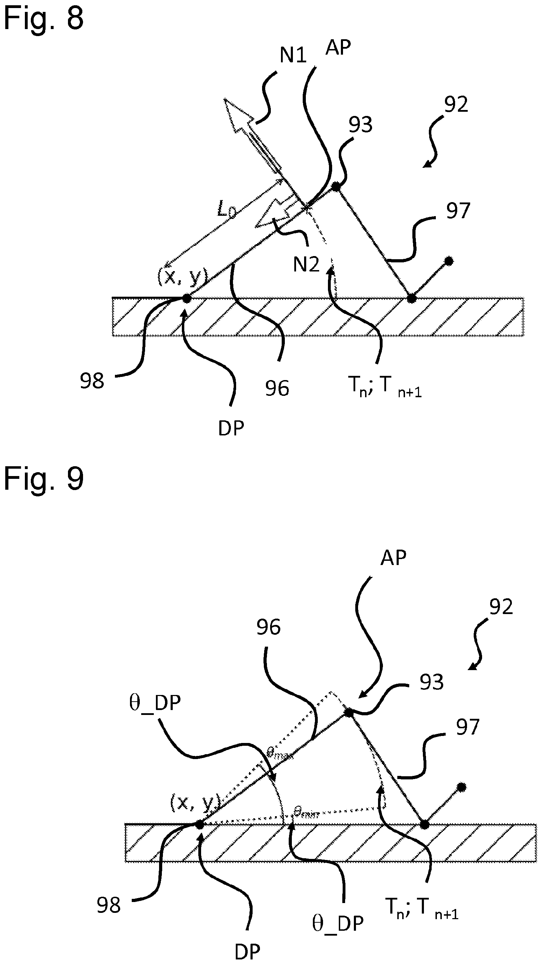

Fig. 8 zeigt entsprechend eine Trajektorie $T_n$; $T_{n+1}$ eines Angriffspunktes AP an einem Oberschenkel 96 eines Patienten 90 mit angepasster Modellfunktion F und die bei einem Anheben eines Beines 92 durch einen Rehabilitationsmechanismus 30 und/oder ein Kniemodul 50 über den Angriffspunkt AP auf das Bein 92 des Patienten 90 ausgeübte Kraft N1, sowie die Einwirkung von Kräften N2 entlang der Achse Angriffspunkt AP - Drehpunkt DP auf das Bein 92 des bettpflichtigen Patienten 90.

In **Fig. 9** sind beispielhaft zwei im Rahmen einer Kurvenanpassung ("curve-fitting") ermittelten Winkel $\theta\_DP$ zwischen jeweils zwei Punkten auf der Trajektorie $T_n$; $T_{n+1}$ und dem Drehpunkt DP, hier speziell einen Winkel $\theta_{max}$ bei maximaler Anhebung des Angriffspunktes AP und einen Winkel $\theta_{min}$ bei minimaler Anhebung des Angriffspunktes AP gezeigt.

Die Differenz der Winkel $\theta_{min}$ und $\theta_{max}$ gibt vorteilhaft Aufschluss über die Mobilität und somit den Therapiefortschritt des Patienten 90.

In den Fig. 4 bis 7c werden beispielhaft verschiedene Ansichten einer Ausgestaltung einer Vorrichtung zur Durchführung des Messverfahrens gezeigt. Zur mechanischen Funktionsweise des Rehabilitationsmechanismus 30 bzw. des Kniemoduls 50 als solches sei vollumfänglich auf die WO 2017/063639 A1 der Anmelderein verwiesen. Die **Fig. 4** und **5** (entsprechen den Fig. 3 und 12 der WO 2017/063639 A1) zeigen beispielhaft einen linken Ausleger 53 eines Kniemoduls 50 eines Rehabilitationsmechanismus 30 einer Vorrichtung zur Durchführung des Messverfahrens in einer perspektivischen Ansicht (Fig. 4) bzw. in einer vergrößerten Seitenansicht (Fig. 5).

In **Fig. 6** (entspricht Fig. 4 der WO 2017/063639 A1) ist ein gesicherter, bettpflichtiger Patienten 90 in einem handelsüblichen Bett nach dem Stand der Technik, insb. Intensivbett, mit angelegtem Knie- 50 und Fußmodul 40 eines Rehabilitationsmechanismus 30 vor einer Vertikalisierung des Bettes in einer perspektivischen Seitenansicht dargestellt.

Die Vorrichtung zur Durchführung des Messverfahrens umfasst wenigstens einen Rehabilitationsmechanismus 30 mit wenigstens einem in Wirkverbindung mit dem Kniegelenk 93 eines Patienten 90 bringbaren Kniemodul 50, umfassend wenigstens einen, oder wie in Fig. 4 dargestellt zwei Elektromotoren 62 zum Antrieb von wenigstens zwei Exzentern 63; 64.

Die Vorrichtung umfasst zudem eine Messeinrichtung 68 zur Aufzeichnung und/oder der Bestimmung einer Trajektorie T des Kniegelenks 93 während der Bewegung des Beins 92, wobei die Messeinrichtung 68 vorzugsweise wenigstens zwei Sensoren 681; 682 zur Bestimmung der Winkelposition der Welle des bzw. der Elektromotoren 62 und/oder der Exzenter 63; 63 umfassen kann.

Darüber hinaus umfasst die Vorrichtung 1 ein Modellgenerierungsmittel 69 zur Erstellung eines kinematischen Modells der Längenverhältnisse, der Position und/oder des Bewegungsradius wenigstens von Teilen der unteren Extremitäten des Patienten 90 aus den von der Messeinrichtung 68 durch Aufzeichnung und/oder der Bestimmung einer Trajektorie T des Kniegelenks 93 während der Bewegung des Beins 92 erhaltenen Daten.

In Fig. 6 ist darüber hinaus ein Steuermodul 60 zur Aussteuerung planmäßiger Rehabilitationsbewegungen zumindest der Gelenke, Muskeln und Sehnen der Beine 92 des bettpflichtigen Patienten 90 mittels des Kniemoduls 50 zu sehen, welches vorzugsweise eingerichtet sein kann, aus den, aus dem kinematischen Modell generierten, patientenspezifischen Daten, Trajektorien T, insbesondere neue, veränderte Trajektorien T, für die Rehabilitationsbewegungen des Kniegelenks 93 zu bestimmen und anhand dieser Trajektorien T das Kniemodul 50, vorzugsweise in Abhängigkeit von Nutzereingaben, auszusteuern.

Das Kniemodul 50 umfasst vorzugsweise wenigstens eine das Kniegelenk 93 des bettpflichtigen Patienten 90 aufnehmende Knie-Orthese 51, ein mit der Knie-Orthese 51 verbundenes Verbindungselement 52;

einen Ausleger 53, an welchem das Verbindungselement 52 befestigt ist, und eine mittels eines Steuermoduls 60 ansteuerbare Mechanik 61, welche über den Ausleger 53 und das Verbindungselement 52 eine definierte Kraft N dergestalt in die Knie-Orthese 51 einleitet, dass über das darin aufgenommene Kniegelenk 93 des bettpflichtigen Patienten 90 die Gelenke, Muskeln und Sehnen dieses Beines 92 planmäßige Rehabilitationsbewegungen ausführen.

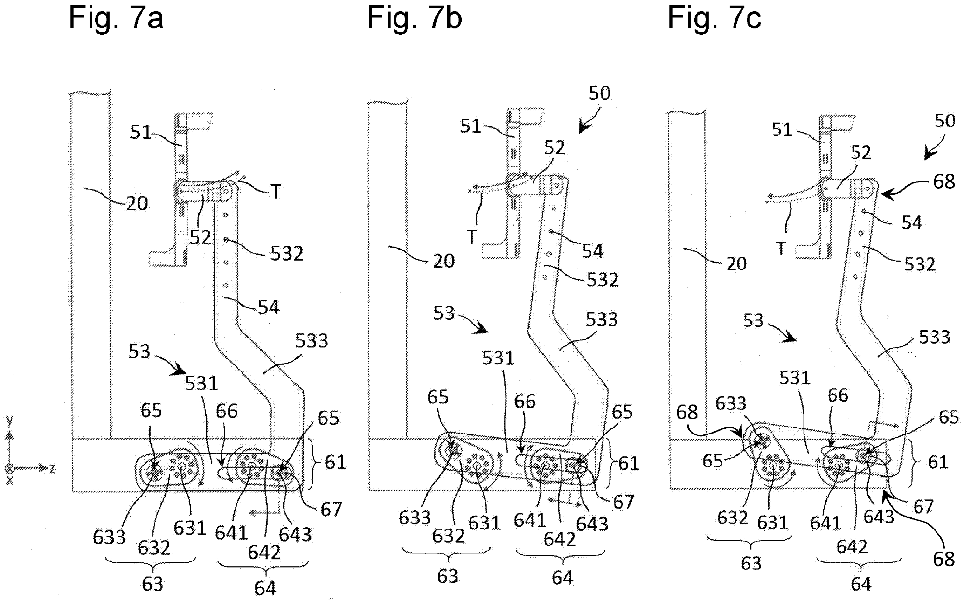

Die **Fig. 7a** bis **7c** veranschaulichen eine von dem Rehabilitationsmechanismus 30 über die Knie-Orthese 51 erzeugte Trajektorie T zum Beugen bzw. Strecken der Beines 92 eines Patienten 90. Die Knie-Orthese 51 kann über das Verbindungselement 52 entweder starr mit dem Ausleger 53 verbunden oder, wie hier zu sehen, beweglich am Ausleger 53 angelenkt sein. Im letzteren Fall, also im Fall der beweglichen Anlenkung und der damit einhergehenden größeren Zahl an Freiheitsgraden, kann zur exakten Aufzeichnung und/oder Bestimmung der Trajektorie T des Kniegelenks 93 vorzugsweise eine weitere Messeinrichtung 68, insbesondere in Form eines Winkelsensors 55 vorgesehen sein.

Schließlich ist in Fig. 5 exemplarisch ein Rehabilitationsmechanismus 30 mit einem Winkelsensor 55 dargestellt, welcher den von einem Verbindungselement 52 zur Knie-Orthese 51 und/oder zum Ausleger 53 eingenommenen Winkel überwacht. Alternativ oder kumulativ dazu kann auch ein Kraftsensor 56, welcher die über den Ausleger 53 und das Verbindungselement 52 in die Knie-Orthese 51 eingeleitete Kraft N überwacht, vorgesehen sein.

Die vorliegende Beschreibung beschreibt ein Messverfahren zur Bestimmung der Längenverhältnisse, der Position und/oder des Bewegungsradius der unteren Extremitäten eines bettpflichtigen Patienten 90, sowie eine Vorrichtung 1 zur Durchführung eines solchen Verfahrens, bei dem wenigstens ein Kniemodul 50 eines Rehabilitationsmechanismus 30 mit einem Kniegelenk 93 des Patienten 90 in Wirkverbindung gebracht wird, mittels einer Messeinrichtung 68, eine Trajektorie T des Kniegelenks 93 während einer Bewegung des Beines 92 mit dem Kniemodul 50 des Rehabilitationsmechanismus 30 aufgezeichnet und/oder bestimmt, und mittels eines Modellgenerierungsmittels 69 aus den so erhaltenen Daten ein kinematisches Modell erstellt wird. Dies ermöglicht vorteilhaft die Bestimmung der Längenverhältnisse, der Position und/oder des Bewegungsradius ohne operativen Eingriff während wenigstens einer initialisierenden Bewegung. Unter Einsparung aufwendiger Sensortechnik stellt die Erfindung vorteilhaft ein kinematisches Modell des Bewegungsradius des Patienten 90 bereit, dessen Parameter als Grundlage zur Erstellung neuer Therapieabläufe innerhalb seiner Bewegungstherapie, sowie als Ausgangsmodell für automatisierte Therapie- und Untersuchungsmethoden dienen können.

## Bezugzeichenliste

| | |
|---|---|
| 10 | Bett, insbesondere: Pflegebett, Krankenbett, Spitalbett oder Intensivbett |
| 11 | Bettrahmen |
| 12 | Längsseiten |
| 13 | Querseiten |
| 14 | Längsseitenbegrenzung |
| 15 | Querseitenbegrenzung |
| 16 | Rollbeine |
| | |
| 20 | Matratze |
| 21 | Matratzenrahmen |
| | |
| 30 | Rehabilitationsmechanismus |
| 32 | am Bett- 11 oder Matratzenrahmen 21 festsetzbare Tragplatte für Rehabilitationsmechanismus 30 |
| | |
| 40 | Fußmodul |
| 41 | Feststellmittel |
| 42 | Trittfläche |
| 43 | Fixierbänder |
| | |
| 50 | Kniemodul |
| 51 | Knie-Orthese |
| 52 | Verbindungselement |
| 53 | Ausleger |
| 531 | Distaler Abschnitt des Auslagers 53 |
| 532 | Proximaler Abschnitt des Auslegers 53 |
| 533 | Zwischenabschnitt des Auslegers 53 |
| 54 | Aufnahmepunkte |
| 55 | Winkelsensor |
| 56 | Kraftsensor |
| 57 | Flügelmutter |
| | |
| 60 | Steuermodul |
| | |
| 61 | Mechanik |
| 62 | Elektromotor |
| | |
| 63 | erster Exzenter |
| 631 | Exzenterwelle des ersten Exzenters 63 |
| 632 | Exzenterscheibe des ersten Exzenters 63 |
| 633 | Steuerbolzen des ersten Exzenters 63 |
| | |
| 64 | zweiter Exzenter |
| 641 | Exzenterwelle des zweiten Exzenters 64 |
| 642 | Exzenterscheibe des zweiten Exzenters 64 |
| 643 | Steuerbolzen des zweiten Exzenters 64 |
| | |
| 65 | Radiallager |
| 66 | Gleitlager |
| 67 | Gleitstein |
| | |
| 68 | Messeinrichtung |
| 681 | Sensor des ersten Exzenters 63 |

682     Sensor des zweiten Exzenters 64
69      Modellgenerierungsmittel

70      Verstellmechanismus

80      Stabilisierungsmechanismus
90      Patient
91      Herz-/Lungen-Thorax
92      Bein
93      Kniegelenk
94      Fuß
95      Fußsohle
96      Oberschenkel
97      Unterschenkel
98      Hüftgelenk

F       Modellfunktion
N1      über den Angriffspunkt AP auf das Bein 92 des Patienten 90 ausgeübte Kraft
N2      Kraft entlang der Achse: Angriffspunkt AP - Drehpunkt DP
$T_n$; $T_{n+1}$   Trajektorien
$L_o$    Länge des Oberschenkels 96
$Y_n$    Y-Koordinaten der Trajektorie T des Kniegelenks 93
$Z_n$    Z-Koordinaten der Trajektorie T des Kniegelenks 93
$t_1, t_2, ..., t_n$   Messzeitpunkt
$Y_H/ Z_H$   Position des Hüftgelenks 98 des bettpflichtigen Patienten 90
AP      Angriffspunkt
DP      Drehpunkt
$\theta\_DP$   Winkel zwischen zwei Punkten auf der Trajektorie ($T_n$; $T_{n+1}$) und dem Drehpunkt (DP)

## Patentansprüche

1. Messverfahren zur Bestimmung der Längenverhältnisse, der Position und/oder des Bewegungsradius der unteren Extremitäten eines bettpflichtigen Patienten (90), wie Hüftgelenk (98), Kniegelenk (93) und/oder Oberschenkel (96), bei dem

   - wenigstens ein Kniemodul (50) eines Rehabilitationsmechanismus (30) über einen Angriffspunkt (AP) mit einem Bein (92) des Patienten (90) in Wirkverbindung gebracht wird;
   - mittels einer Messeinrichtung (68), eine Trajektorie ($T_n$; $T_{n+1}$) allein des Angriffspunkts (AP) während einer Bewegung des Beines (92) mit dem Kniemodul (50) des Rehabilitationsmechanismus (30) aufzeichnet und/oder bestimmt wird; und
   - mittels eines Modellgenerierungsmittels (69) aus den so erhaltenen Daten ein kinematisches Modell der Längenverhältnisse, der Position und/oder des Bewegungsradius wenigstens von Teilen der unteren Extremitäten des Patienten (90) erstellt wird; wobei
   - in wenigstens einem Verfahrensschritt ermittelte Koordinatenpaare ($Y_1$/ $Z_1$, $Y_2$/ $Z_2$, ..., $Y_n$/ $Z_n$) oder Koordinatentripel ($Y_1$ / $Z_1$/ $t_1$; $Y_2$ / $Z_2$/ $t_2$; .../...; $Y_n$ / $Z_n$/ $t_n$) mit einer Modelfunktion in Form einer Koordinatengleichung verglichen und dadurch im Rahmen einer Kurvenanpassung ("curve-fitting") die Position ($Y_H(t)$ / $Z_H(t)$) eines Drehpunkts (DP) und/oder die Länge ($L_o$) zwischen Angriffspunkt (AP) und Drehpunkt (DP) des bettpflichtigen Patienten (90), sowie ein Winkel ($\theta\_DP$) zwischen zwei Punkten auf der Trajektorie ($T_n$; $T_{n+1}$) und dem Drehpunkt (DP) ermittelt werden; wobei

   - als Modelfunktion die Koordinatengleichung $$\frac{(Y_n - M_Y(Y_H))^2}{L_0{}^2} + \frac{(Z_n - M_Z(Z_H))^2}{(L_0 \pm \Delta L)^2} = 1$$ für eine Ellipse;
   - als Parameter für den Mittelpunkt der Ellipse eine Funktion ($M_Y(Y_H)$; $M_Z(Z_H)$) in Abhängigkeit von der Position ($Y_H(t)$ / $Z_H(t)$) des Drehpunkts (DP); und
   - als große Halbachse der Ellipse die Länge ($L_o$) zwischen Angriffspunkt (AP) und Drehpunkt (DP) des bettpflichtigen Patienten (90), wobei der Term ($L_o \pm \Delta L$) eine mögliche Verschiebung der Position ($Y_H(t)/Z_H(t)$) des Drehpunkts (DP) während einer Bewegung beschreibt,

zugrunde gelegt wird.

2. Messverfahren nach Anspruch 1, bei dem der Angriffspunkt (AP) das Kniegelenk (93) des Patienten (90) ist.

3. Messverfahren nach Anspruch 1 oder 2, bei dem in einem Verfahrensschritt das Bein (92) über einen Angriffspunkt (AP), insbesondere über das Kniegelenk (93), den Oberschenkel (96) und/oder einen Unterschenkel (97) mittels einer Knie-Orthese (51) am Kniemodul (50) fixiert wird.

4. Messverfahren nach einem der Ansprüche 1 bis 3, bei dem in einem Verfahrensschritt die Messeinrichtung (68) ausgehend von einer Ausgangposition (Po) eine manuell und/oder automatisiert geführte Bewegung des Beins (92), insbesondere als Trajektorie ($T_n$; $T_{n+1}$) eines Angriffspunkts (AP), insbesondere des Kniegelenks (93), in Form von Koordinatenpaaren ($Y_1$/ $Z_1$, $Y_2$/ $Z_2$, ..., $Y_n$/ $Z_n$) oder zeitabhängig in Form von Koordinatentripeln ($Y_1$/ $Z_1$/ $t_1$, $Y_2$/ $Z_2$/ $t_2$, ..., $Y_n$/ $Z_n$/ $t_n$), aufzeichnet.

**5.** Messverfahren nach einem oder mehreren der vorherigen Ansprüche, bei dem die Messeinrichtung (68) die Trajektorie ($T_n$; $T_{n+1}$) des Angriffspunkts (AP), insbesondere des Kniegelenks (93), in Form von Koordinatenpaaren ($Y_1$/ $Z_1$, $Y_2$/ $Z_2$, ..., $Y_n$/ $Z_n$) oder zeitabhängig in Form von Koordinatentripeln ($Y_1$/ $Z_1$/ $t_1$, $Y_2$/ $Z_2$/ $t_2$, ..., $Y_n$/ $Z_n$/ $t_n$), mit Hilfe von wenigstens zwei Sensoren (681; 682) aus den Winkelpositionen von elektromotorisch angetriebenen Exzentern (63; 64) bestimmt.

**6.** Messverfahren nach einem oder mehreren der vorherigen Ansprüche,

- bei dem die Position ($Y_H(t)/Z_H(t)$) eines Drehpunkts (DP) die Position des Hüftgelenks (98) des bettpflichtigen Patienten (90) ist; und/oder
- bei dem die Länge ($L_o$) zwischen Angriffspunkt (AP) und Drehpunkt (DP) die Länge des Oberschenkels (96) des bettpflichtigen Patienten (90) ist.

**7.** Messverfahren nach einem oder mehreren der vorherigen Ansprüche, bei dem in einem Verfahrensschritt ein Steuermodul (60) zur Aussteuerung planmäßiger Rehabilitationsbewegungen zumindest der Gelenke, Muskeln und Sehnen der Beine (92) des bettpflichtigen Patienten (90) mittels des Kniemoduls (50) vorgesehen ist, wobei das Steuermodul (60) vorzugsweise

- aus den, aus dem kinematischen Modell generierten, patientenspezifischen Daten, Trajektorien ($T_n$; $T_{n+1}$), insbesondere neue, veränderte Trajektorien ($T_n$; $T_{n+1}$), für die Rehabilitationsbewegungen des Beines (92) eines Patienten (90) bezüglich des Angriffspunkts (AP), insbesondere des Kniegelenks (93), bestimmt und
- anhand dieser Trajektorien ($T_n$; $T_{n+1}$) das Kniemodul (50), vorzugsweise in Abhängigkeit von Nutzereingaben, aussteuert.

**8.** Messverfahren nach einem oder mehreren der vorherigen Ansprüche, bei dem der Rehabilitationsmechanismus (30) und/oder das Kniemodul (50) während der manuell und/oder automatisiert geführten Bewegung des Beins (92) mit Hilfe des Steuermoduls (60) derart gesteuert wird, dass die Einwirkung von Kräften (N2) entlang einer durch Angriffspunkt (AP) und Drehpunkt (DP) definierten Achse durch den Rehabilitationsmechanismus (30) und/oder das Kniemodul (50) auf das Bein (92) des bettpflichtigen Patienten (90) vermieden ist.

**9.** Messverfahren nach Anspruch 7 oder 8, bei dem das Steuermodul (60) aufgrund berechneter Trajektorien ($T_n$; $T_{n+1}$) für den Angriffspunkt (AP), insbesondere für das Kniegelenk (93), das Kniemodul (50) und/oder den Rehabilitationsmechanismus (30) derart steuert, dass der Angriffspunkt (AP), insbesondere das Kniegelenk (93), des bettpflichtigen Patienten (90) mit Hilfe des Kniemoduls (50) und ggf. der Knie-Orthese (51) entlang dieser, insbesondere neuen, veränderten, Trajektorien ($T_n$; $T_{n+1}$) bewegt wird.

**10.** Messverfahren nach einem der Ansprüche 7 bis 9, bei dem in einem Verfahrensschritt nach jeder automatisiert geführten Bewegung des Beins (92), aus jeder neuen, zeitabhängig aufgezeichneten Trajektorie ($T_{n+1}$) des Angriffspunkts (AP) neue Werte für

- die Länge ($L_o$) zwischen Angriffspunkt (AP) und Drehpunkt (DP),
- die Position ($Y_H(t)$ / $Z_H(t)$) des Drehpunkts (DP),
- sowie den Winkel ($\theta\_DP$) zwischen zwei Punkten auf der Trajektorie ($T_{n+1}$) und dem Drehpunkt (DP)

berechnet und mit den korrespondierenden Werten zuvor gemessener Trajektorien ($T_n$) verglichen werden.

**11.** Messverfahren nach einem der Ansprüche 7 bis 10, bei dem in einem Verfahrensschritt, anhand des Vergleichs der Werte für

- die Länge ($L_o$) zwischen Angriffspunkt (AP) und Drehpunkt (DP),
- die Position ($Y_H(t)$ / $Z_H(t)$) des Drehpunkts (DP),
- sowie den Winkel zwischen zwei Punkten auf der Trajektorie ($T_n$; $T_{n+1}$) und dem Drehpunkt (DP)

von je zwei, zeitlich aufeinander folgend gemessenen Trajektorien ($T_n$, $T_{n+1}$) mittels eines Optimierungs-Algorithmus, insbesondere eines *Least-Squares*-Algorithmus, ein aktualisiertes Modell mit verbesserten Werten für
- die Länge ($L_o$) zwischen Angriffspunkt (AP) und Drehpunkt (DP),
- die Position ($Y_H(t)$ / $Z_H(t)$) des Drehpunkts (DP),
- sowie einen Winkel ($\theta\_DP$) zwischen zwei Punkten auf der Trajektorie ($T_n$; $T_{n+1}$) und dem Drehpunkt (DP)

erzeugt wird.

**12.** Messverfahren nach einem der Ansprüche 7 bis 11, bei dem in einem Verfahrensschritt die Werte des aktualisierten Modells als Basis für eine weitere, automatisiert geführte Bewegung des Beins (92) dienen.

**13.** Vorrichtung (1) zur Durchführung eines Messverfahrens zur Bestimmung der Längenverhältnisse, der Position und/oder des Bewegungsradius der unteren Extremitäten eines bettpflichtigen Patienten (90), wie Hüftgelenk (98), Kniegelenk (93) und/oder Oberschenkel (96) nach einem der Ansprüche 1 bis 12, wenigstens umfassend

- einen Rehabilitationsmechanismus (30) mit wenigstens einem in Wirkver-bindung mit über einen Angriffspunkt (AP) mit einem Bein (92) eines Patienten (90) bringbaren Kniemodul (50), umfassend wenigstens einen Elektromotor (62) zum Antrieb von wenigstens zwei, am Kniemodul (50) angeordneten Exzentern (63; 64),
- eine Messeinrichtung (68) eingerichtet zur Aufzeichnung und/oder der Bestimmung einer Trajektorie ($T_n$; $T_{n+1}$) allein des Angriffspunktes (AP), insbesondere des Kniegelenks (93), während der Bewegung des Beins (92); und
- ein Modellgenerierungsmittel (69) eingerichtet zur Erstellung eines kinematischen Modells der Längenverhältnisse, der Position und/oder des Bewegungsradius wenigstens von Teilen der unteren Extremitäten des Patienten (90) aus den so erhaltenen Dater entsprechend dem durch das Modellgenerierungsmittel im Anspruch 1 durchgeführten Verfahren.

14. Vorrichtung (1) nach Anspruch 13, **dadurch gekennzeichnet,**

- **dass** die Messeinrichtung (68) wenigstens zwei Sensoren (681; 682) zur Bestimmung der Winkelposition der Welle des Elektromotors (62) und/oder der Exzenter (63; 64) umfasst;
- und/oder dass ein Steuermodul (60) zur Aussteuerung planmäßiger Rehabilitationsbewegungen zumindest der Gelenke, Muskeln und Sehnen der Beine (92) des bettpflichtigen Patienten (90) mittels des Kniemoduls (50) vorgesehen ist, wobei das Steuermodul (60) vorzugsweise eingerichtet ist, aus den, aus dem kinematischen Modell generierten, patientenspezifischen Daten, Trajektorien ($T_n$; $T_{n+1}$), insbesondere neue, veränderte Trajektorien ($T_n$; $T_{n+1}$), für die Rehabilitationsbewegungen des Beines (92) eines Patienten (90) bezüglich des Angriffspunkts (AP), insbesondere des Kniegelenks (93), zu bestimmen und anhand dieser Trajektorien ($T_n$; $T_{n+1}$) das Kniemodul (50), vorzugsweise in Abhängigkeit von Nutzereingaben, auszusteuern.

15. Vorrichtung (1) nach Anspruch 13 oder 14, **dadurch gekennzeichnet,**

- **dass** das Kniemodul (50)
- wenigstens eine das Kniegelenk (93) des bettpflichtigen Patienten (90) aufnehmende Knie-Orthese (51);
- ein mit der Knie-Orthese (51) verbundenes Verbindungselement (52);
- einen Ausleger (53), an welchem das Verbindungselement (52) befestigt ist; und
- eine mittels eines Steuermoduls (60) ansteuerbare Mechanik (61) umfasst; wobei die Mechanik (61) über den Ausleger (53) und das Verbindungselement (52) eine definierte Kraft (N) dergestalt in die Knie-Orthese (51) einleitet, dass über den Angriffspunkt (AP), insbesondere das darin aufgenommene Kniegelenk (93) des bettpflichtigen Patienten (90), die Gelenke, Muskeln und Sehnen dieses Beines (92) planmäßige Rehabilitationsbewegungen ausführen;
- und/oder dass der Rehabilitationsmechanismus (30)
- wenigstens einen Winkelsensor (55), welcher den von einem Verbindungselement (52) zur Knie-Orthese (51) und/oder zu einem Ausleger (53) eingenommenen Winkel überwacht; und/oder
- einen Kraftsensor (56), welcher die über den Ausleger (53) und das Verbindungselement (52) in die Knie-Orthese (51) eingeleitete Kraft (N) überwacht, umfasst.

**Claims**

1. Measuring method for determining the length conditions, the position and/or the radius of movement of the lower extremities of a bedridden patient (90), such as hip joint (98), knee joint (93) and/or upper leg (96), in which method

- at least one knee module (50) of a rehabilitation mechanism (30) is operatively connected to a leg (92) of the patient (90) via an application point (AP);
- a measuring device (68) is used for recording and/or determining a trajectory ($T_n$; $T_{n+1}$) solely of the application point (AP) during a movement of the leg (92) with the knee module (50) of the rehabilitation mechanism (30); and
- a model-generating means (69) is used to create, from the data thus obtained, a kinematic model of the length conditions, the position and/or the radius of movement of at least parts of the lower extremities of the patient (90); wherein,
- in at least one method step, determined coordinate pairs ($Y_1/Z_1$, $Y_2/Z_2$, ..., $Y_n/Z_n$) or coordinate triplets ($Y_1/Z_1/t_1$; $Y_2/Z_2/t_2$; .../...; $Y_n/Z_n/t_n$) are compared with a model function in the form of a coordinate equation, and in this way, in the context of a curve-fitting, the position ($Y_H(t)/Z_H(t)$) of a rotation point (DP) and/or the

length ($L_0$) between application point (AP) and rotation point (DP) of the bedridden patient (90), and an angle ($\theta\_DP$) between two points on the trajectory ($T_n$; $T_{n+1}$) and the rotation point (DP) are determined; wherein the following are used:

- as model function, in particular the coordinate equation

$$\frac{(Y_n - M_Y(Y_H))^2}{L_0{}^2} + \frac{(Z_n - M_Z(Z_H))^2}{(L_0 \pm \Delta L)^2} = 1 \text{ for}$$

an ellipse;
- as parameter for the center point of the ellipse, a function ($M_Y(Y_H)$; $M_Z(Z_H)$) according to the position ($Y_H(t)$ / $Z_H(t)$) of the rotation point (DP); and
- as semi-major axis of the ellipse, the length ($L_0$) between application point (AP) and rotation point (DP) of the bedridden patient (90), wherein the term ($L_0 \pm \Delta L$) describes a possible shift of the position ($Y_H(t)$ /$Z_H(t)$) of the rotation point (DP) during a movement.

2. Measuring method according to Claim 1, in which the application point (AP) is the knee joint (93) of the patient (90).

3. Measuring method according to Claim 1 or 2, in which, in one method step, the leg (92) is secured to the knee module (50) via an application point (AP), in particular via the knee joint (93), the upper leg (96) and/or a lower leg (97), by means of a knee orthosis (51).

4. Measuring method according to one of Claims 1 to 3, in which, in one method step, the measuring device (68), proceeding from a starting position ($P_0$), records a manually and/or automatically guided movement of the leg (92), in particular as a trajectory ($T_n$; $T_{n+1}$) of an application point (AP), in particular of the knee joint (93), in the form of coordinate pairs ($Y_1$/ $Z_1$, $Y_2$/ $Z_2$, ..., $Y_n$/ $Z_n$) or time-dependently in the form of coordinate triplets ($Y_1$/ $Z_1$/ $t_1$, $Y_2$/ $Z_2$/ $t_2$, ..., $Y_n$/ $Z_n$/ $t_n$) .

5. Measuring method according to one or more of the preceding claims, in which the measuring device (68) is determined the trajectory ($T_n$; $T_{n+1}$) of the application point (AP), in particular of the knee joint (93), in the form of coordinate pairs ($Y_1$/$Z_1$, $Y_2$/ $Z_2$, ..., $Y_n$/ $Z_n$) or time-dependently in the form of coordinate triplets ($Y_1$/ $Z_1$/ $t_1$, $Y_2$/ $Z_2$/ $t_2$, ..., $Y_n$/ $Z_n$/ $t_n$), with the aid of at least two sensors (681; 682) from the angle positions of eccentrics (63; 64) that are driven by electric motor.

6. Measuring method according to one or more of the preceding claims,

- in which the position ($Y_H(t)$ / $Z_H(t)$) of a rotation point (DP) is the position of the hip joint (98) of the bedridden patient (90); and/or
- in which the length ($L_0$) between application point (AP) and rotation point (DP) is the length of the upper leg (96) of the bedridden patient (90).

7. Measuring method according to one or more of the preceding claims, in which, in one method step, a control module (60) is provided for controlling planned rehabilitation movements at least of the joints, muscles and tendons of the legs (92) of the bedridden patient (90) by means of the knee module (50), wherein the control module (60) preferably

- determines, from the patient-specific data generated from the kinematic model, trajectories ($T_n$; $T_{n+1}$), in particular new, modified trajectories ($T_n$; $T_{n+1}$), for the rehabilitation movements of the leg (92) of a patient (90) with respect to the application point (AP), in particular the knee joint (93), and,
- on the basis of these trajectories ($T_n$; $T_{n+1}$), controls the knee module (50), preferably according to user inputs.

8. Measuring method according to one or more of the preceding claims, in which the rehabilitation mechanism (30) and/or the knee module (50), during the manually and/or automatically guided movement of the leg (92), is controlled with the aid of the control module (60) in such a way that the effect of forces (N2) along an axis, defined by application point (AP) and rotation point (DP), through the rehabilitation mechanism (30) and/or the knee module (50) on the leg (92) of the bedridden patient (90) is avoided.

9. Measuring method according to Claim 7 or 8, in which the control module (60), on the basis of calculated trajectories ($T_n$; $T_{n+1}$) for the application point (AP), in particular for the knee joint (93), controls the knee module (50) and/or the rehabilitation mechanism (30) in such a way that the application point (AP), in particular the knee joint (93), of the bedridden patient (90), is moved along these new, modified trajectories ($T_n$; $T_{n+1}$) with the aid of the knee module (50) and optionally of the knee orthosis (51).

10. Measuring method according to one of Claims 7 to 9, in which, in one method step, after each automatically guided movement of the leg (92), new values are calculated, from each new time-dependently recorded trajectory ($T_{n+1}$) of the application points (AP), for

- the length ($L_0$) between application point (AP) and rotation point (DP),

- the position ($Y_H(t)$ / $Z_H(t)$) of the rotation point (DP),
- and the angle ($\theta\_DP$) between two points on the trajectory ($T_{n+1}$) and the rotation point (DP),

and these new values are compared with the corresponding values of previously measured trajectories ($T_n$).

**11.** Measuring method according to one of Claims 7 to 10, in which, in one method step, on the basis of the comparison of the values for

- the length ($L_o$) between application point (AP) and rotation point (DP),
- the position ($Y_H(t)$ / $Z_H(t)$) of the rotation point,
- and the angle between two points on the trajectory ($T_n$; $T_{n+1}$) and the rotation point (DP),
in each case of two successively measured trajectories ($T_n$, $T_{n+1}$), an updated model with improved values for
- the length ($L_o$) between application point (AP) and rotation point (DP),
- the position ($Y_H(t)$ / $Z_H(t)$) of the rotation point (DP),
- and an angle ($\theta\_DP$) between the two points on the trajectory ($T_n$; $T_{n+1}$) and the rotation point (DP)

is generated by means of an optimization algorithm, in particular a *least squares* algorithm.

**12.** Measuring method according to one of Claims 7 to 11, in which, in one method step, the values of the updated model serve as a basis for a further automatically guided movement of the leg (92).

**13.** Apparatus (1) for carrying out a measuring method for determining the length conditions, the position and/or the radius of movement of the lower extremities of a bedridden patient (90), such as hip joint (98), knee joint (93) and/or upper leg (96), according to one of Claims 1 to 12, at least comprising

- a rehabilitation mechanism (30) with at least one knee module (50) which can be operatively connected via an application point (AP) to a leg (92) of a patient (90) and which comprises at least one electric motor (62) for driving at least two eccentrics (63; 64) arranged on the knee module (50),
- a measuring device (68) configured for recording and/or determining a trajectory ($T_n$; $T_{n+1}$) solely of the application point (AP), in particular of the knee joint (93), during the movement of the leg (92); and
- a model-generating means (69) configured for creating, from the data thus obtained, a kinematic model of the length conditions, the position and/or the radius of movement of at least parts of the lower extremities of the patient (90) in accordance with the method carried out by the model-generating means in Claim 1.

**14.** Apparatus (1) according to Claim 13, **characterized**

- **in that** the measuring device (68) comprises at least two sensors (681; 682) for determining the angle position of the shaft of the electric motor (62) and/or of the eccentrics (63; 64);
- and/or in that a control module (60) for controlling planned rehabilitation movements at least of the joints, muscles and tendons of the legs (92) of the bedridden patient (90) by means of the knee module (50) is provided, wherein the control module (60) is preferably configured to determine, from the patient-specific data generated from the kinematic model, trajectories ($T_n$; $T_{n+1}$), in particular new, modified trajectories ($T_n$; $T_{n+1}$), for the rehabilitation movements of the leg (92) of a patient (90) with respect to the application point (AP), in particular the knee joint (93), and, on the basis of these trajectories ($T_n$; $T_{n+1}$), to control the knee module (50), preferably according to user inputs.

**15.** Apparatus (1) according to Claim 13 or 14, **characterized**

- **in that** the knee module (50) comprises

- at least one knee orthosis (51) receiving the knee joint (93) of the bedridden patient (90);
- a connection element (52) connected to the knee orthosis (51);
- an extension arm (53) on which the connection element (52) is secured; and
- a mechanical device (61) that can be driven by means of a control module (60); wherein the mechanical device (61) introduces a defined force (N) into the knee orthosis (51), via the extension arm (53) and the connection element (52), in such a way that the joints, muscles and tendons of this leg (92) perform planned rehabilitation movements via the application point (AP), in particular via the therein received knee joint (93) of the bedridden patient (90);

- and/or in that the rehabilitation mechanism (30) comprises

- at least one angle sensor (55) which monitors the angle adopted by a connection element (52) to the knee orthosis (51) and/or to an extension arm (53); and/or
- a force sensor (56) which monitors the force (N) introduced into the knee orthosis (51) via the extension arm (53) and the connection element (52).

## Revendications

1. Procédé de mesure permettant de déterminer les rapports de longueur, la position et/ou le rayon de déplacement des extrémités inférieures d'un patient alité (90), par exemple une articulation de la hanche (98), une articulation du genou (93) et/ou un fémur (96), dans lequel :

 - au moins un module de genou (50) d'un mécanisme de réhabilitation (30) est amené en liaison active avec une jambe (92) du patient (90) via un point d'application (AP) ;
 - une trajectoire ($T_n$ ; $T_{n+1}$) du seul point d'application (AP) pendant un mouvement de la jambe (92) avec le module de genou (50) du mécanisme de réhabilitation (30) est désignée et/ou est déterminée au moyen d'un dispositif de mesure (68) ; et
 - un modèle cinématique des rapports de longueur, de la position et/ou du rayon de déplacement est établi au moins par des parties des extrémités inférieures du patient (90) à l'aide d'un moyen de génération de modèle (69) réalisé à partir des données ainsi obtenues ;
 - au cours d'au moins une étape de procédé, les paires de coordonnées ($Y_1 / Z_1$, $Y_2 / Z_2$, ..., $Y_n / Z_n$) ou les triplets de coordonnées ($Y_1 / Z_1 / t_1$ ; $Y_2 / Z_2 / t_2$ ; .../... ; $Y_n / Z_n / t_n$) déterminés sont comparés à une fonction de modèle prenant la forme d'une équation de coordonnées, permettant ainsi de déterminer dans le cadre d'un ajustement de courbe (« curve-fitting ») la position ($Y_H(t) / Z_H(t)$) d'un point de rotation (DP) et/ou la longueur ($L_o$) entre le point d'application (AP) et le point de rotation (DP) du patient alité (90), ainsi qu'un angle ($\theta\_DP$) entre deux points sur la trajectoire ($T_n$ ; $T_{n+1}$) et le point de rotation (DP) ;

 dans lequel on prend pour base :

 - l'équation de coordonnées servant de fonction de modèle
$$\frac{(Y_n - M_Y(Y_H))^2}{L_0{}^2} + \frac{(Z_n - M_Z(Z_H))^2}{(L_0 \pm \Delta L)^2} = 1 \quad \text{pour}$$
une ellipse ;
 - comme paramètre pour le point médian de l'ellipse, une fonction ($M_Y(Y_H)$ ; $M_Z(Z_H)$) en fonction de la position ($Y_H(t) / Z_H(t)$) du point de rotation (DP) ; et
 - comme grand demi-axe de l'ellipse, la longueur ($L_0$) entre le point d'application (AP) et le point de rotation (DP) du patient alité (90), le terme ($L_0 \pm \Delta L$) décrivant un décalage possible de la position ($Y_H(t) / Z_H(t)$) du point de rotation (DP) pendant un mouvement.

2. Procédé de mesure selon la revendication 1, dans lequel le point d'application (AP) est l'articulation du genou (93) du patient (90).

3. Procédé de mesure selon la revendication 1 ou 2, dans lequel au cours d'une étape de procédé, la jambe (92) est fixée au niveau du module de genou (50) via un point d'application (AP), notamment via l'articulation du genou (93), le fémur (96) et/ou un bas de la jambe (97), au moyen d'une orthèse de genou (51).

4. Procédé de mesure selon l'une quelconque des revendications 1 à 3, dans lequel au cours d'une étape de procédé, le dispositif de mesure (68) désigne, à partir d'une position de départ ($P_0$), un mouvement, réalisé manuellement et/ou de façon automatisée, de la jambe (92), notamment sous la forme d'une trajectoire ($T_n$ ; $T_{n+1}$) d'un point d'application (AP), notamment de l'articulation du genou (93), sous la forme de paires de coordonnées ($Y_1 / Z_1$, $Y_2 / Z_2$, ..., $Y_n / Z_n$) ou en fonction du temps sous la forme de triplets de coordonnées ($Y_1 / Z_1 / t_1$, $Y_2 / Z_2 / t_2$, ..., $Y_n / Z_n / t_n$).

5. Procédé de mesure selon au moins l'une quelconque des revendications précédentes ou plusieurs d'entre elles, dans lequel le dispositif de mesure (68) est déterminé la trajectoire ($T_n$ ; $T_{n+1}$) du point d'application (AP), notamment de l'articulation du genou (93), sous la forme de paires de coordonnées ($Y_1 / Z_1$, $Y_2 / Z_2$, ..., $Y_n / Z_n$) ou en fonction du temps sous la forme de triplets de coordonnées ($Y_1 / Z_1 / t_1$, $Y_2 / Z_2 / t_2$, ..., $Y_n / Z_n / t_n$), à l'aide d'au moins deux capteurs (681 ; 682) en partant des positions angulaires depuis les excentres (63 ; 64) entraînés de façon électromotorisée.

6. Procédé de mesure selon au moins l'une quelconque des revendications précédentes ou plusieurs d'entre elles,

 - dans lequel la position ($Y_H(t) / Z_H(t)$) d'un point de rotation (DP) est la position de l'articulation de la hanche (98) du patient alité (90) ; et/ou
 - dans lequel la longueur ($L_0$) entre le point d'application (AP) et le point de rotation (DP) est la longueur du fémur (96) du patient alité (90).

7. Procédé de mesure selon au moins l'une quelconque des revendications précédentes ou plusieurs d'entre elles, dans lequel au cours d'une étape de procédé, un module de commande (60) est prévu pour régler des mouvements de réhabilitation programmés au moins des articulations, des muscles et des tendons des jambes (92) du patient alité (90) à l'aide du module de genou (50), le module de commande (60) :

- définissant de préférence à partir des données spécifiques au patient générées à partir du modèle cinématique, des trajectoires ($T_n$ ; $T_{n+1}$) notamment de nouvelles trajectoires modifiées ($T_n$ ; $T_{n+1}$) pour les mouvements de réhabilitation de la jambe (92) d'un patient (90) par rapport au point d'application (AP), notamment l'articulation du genou (93) ; et
- réglant de préférence à l'aide de ces trajectoires ($T_n$ ; $T_{n+1}$) le module de genou (50), de préférence en fonction des entrées saisies par l'utilisateur.

8. Procédé de mesure selon au moins l'une quelconque des revendications précédentes ou plusieurs d'entre elles, dans lequel le mécanisme de réhabilitation (30) et/ou le module de genou (50) est commandé de telle sorte pendant le mouvement, réalisé manuellement et/ou de façon automatisée, de la jambe (92) à l'aide du module de commande (60), que l'effet des forces (N2) est évité le long d'un axe défini par le point d'application (AP) et le point de rotation (DP) par le biais du mécanisme de réhabilitation (30) et/ou du module de genou (50) sur la jambe (92) du patient alité (90) .

9. Procédé de mesure selon la revendication 7 ou 8, dans lequel le module de commande (60) commande sur la base des trajectoires ($T_n$ ; $T_{n+1}$) calculées pour le point d'application (AP), notamment pour l'articulation du genou (93), le module de genou (50) et/ou le mécanisme de réhabilitation (30) de telle sorte que le point d'application (AP), notamment l'articulation du genou (93), du patient alité (90) soit déplacé à l'aide du module de genou (50) et le cas échéant de l'orthèse de genou (51) le long de celle-ci, notamment le long de nouvelles trajectoires modifiées ($T_n$ ; $T_{n+1}$).

10. Procédé de mesure selon l'une quelconque des revendications 7 à 9, dans lequel au cours d'une étape de procédé selon chaque mouvement, réalisé de façon automatisée, de la jambe (92), à partir de chaque nouvelle trajectoire ($T_{n+1}$) dessinée, en fonction du temps, du point d'application (AP), de nouvelles valeurs pour :

- la longueur ($L_o$) entre le point d'application (AP) et le point de rotation (DP) ;

- la position ($Y_H(t)$ / $Z_H(t)$) du point de rotation (DP) ;
- ainsi que l'angle ($\theta\_DP$) entre deux points sur la trajectoire ($T_{n+1}$) et le point de rotation (DP) ;

sont calculées et comparées aux valeurs correspondantes des trajectoires ($T_n$) précédemment mesurées.

11. Procédé de mesure selon l'une quelconque des revendications 7 à 10, dans lequel au cours d'une étape de procédé, à l'aide de la comparaison des valeurs pour :

- la longueur ($L_o$) entre le point d'application (AP) et le point de rotation (DP) ;
- la position ($Y_H(t)$ / $Z_H(t)$) du point de rotation (DP) ;
- ainsi que l'angle entre deux points sur la trajectoire ($T_n$ ; $T_{n+1}$) et le point de rotation (DP) ;
à partir de respectivement deux trajectoires ($T_n$, $T_{n+1}$) mesurées à la suite l'une de l'autre dans le temps, un modèle actualisé avec des valeurs améliorées pour :
- la longueur ($L_o$) entre le point d'application (AP) et le point de rotation (DP) ;
- la position ($Y_H(t)$ / $Z_H(t)$) du point de rotation (DP) ;
- ainsi qu'un angle ($\theta\_DP$)entre deux points sur la trajectoire ($T_n$ ; $T_{n+1}$) et le point de rotation (DP) ;

est produit à l'aide d'un algorithme d'optimisation, notamment d'un algorithme des moindres carrés.

12. Procédé de mesure selon l'une quelconque des revendications 7 à 11, dans lequel au cours d'une étape de procédé, les valeurs du modèle actualisé servent de base pour un autre mouvement, réalisé de façon automatisée, de la jambe (92).

13. Dispositif (1) de mise en œuvre d'un procédé de mesure permettant de déterminer les rapports de longueur, la position et/ou le rayon de déplacement des extrémités inférieures d'un patient alité (90), par exemple une articulation de la hanche (98), une articulation du genou (93) et/ou un fémur (96) selon l'une quelconque des revendications 1 à 12 ; comprenant au moins :

- un mécanisme de réhabilitation (30) avec au moins un module de genou (50) pouvant être amené en liaison active avec une jambe (92) d'un patient (90) via un point d'application (AP), comprenant au moins un moteur électrique (62) pour l'entraînement d'au moins deux excentres (63 ; 64) disposés au niveau du module de genou (50) ;

- un dispositif de mesure (68) conçu pour le traçage et/ou la détermination d'une trajectoire ($T_n$ ; $T_{n+1}$) du seul point d'application (AP), notamment de l'articulation du genou (93), pendant le mouvement de la jambe 92) ; et

- un moyen de génération de modèle (69) conçu pour établir un modèle cinématique des rapports de longueur, de la position et/ou du rayon de déplacement au moins par des parties des extrémités inférieures du patient (90) à partir des données ainsi obtenues en fonction du procédé mis en œuvre dans la revendication 1 par le moyen de génération de modèle.

14. Dispositif (1) selon la revendication 13, **caractérisé en ce que** :

- le dispositif de mesure (68) comprend au moins deux capteurs (681 ; 682) permettant de déterminer la position angulaire de l'arbre du moteur électrique (62) et/ou des excentres (63 ; 64) ; et/ou

- un module de commande (60) est prévu pour déterminer des mouvements de réhabilitation programmés au moins pour les articulations, les muscles et les tendons des jambes (92) du patient alité (90) à l'aide du module de genou (50), le module de commande (60) étant de préférence conçu pour régler, à partir des données spécifiques au patient générées à partir du modèle cinématique, des trajectoires ($T_n$ ; $T_{n+1}$), notamment de nouvelles trajectoires modifiées ($T_n$ ; $T_{n+1}$), pour les mouvements de réhabilitation de la jambe (92) d'un patient (90) par rapport au point d'application (AP), notamment de l'articulation du genou (93) et pour commander, à l'aide de ces trajectoires ($T_n$ ; $T_{n+1}$) le module de genou (50), de préférence en fonction des entrées saisies par l'utilisateur.

15. Dispositif (1) selon la revendication 13 ou 14, **caractérisé en ce que** :

- le module de genou (50) comprend :
- au moins une orthèse de genou (51) recevant l'articulation du genou (93) du patient alité (90) ;
- un élément de liaison (52) relié à l'orthèse de genou (51) ;
- une potence (53) à laquelle l'élément de liaison (52) est fixé ; et
- un mécanisme (61) pouvant être commandé à l'aide d'un module de commande (60) ;

le mécanisme (61) introduisant de telle sorte une force (N) définie dans l'orthèse de genou (51), via la potence (53) et l'élément de liaison (52), que les articulations, les muscles et les tendons de cette jambe (92) réalisent des mouvements de réhabilitation programmés via le point d'application (AP), notamment l'articulation du genou (93), logée à l'intérieur, du patient alité (90) ;

et/ou que le mécanisme de réhabilitation (30) comprend :

- au moins un capteur angulaire (55) qui surveille l'angle formé par un élément de liaison (52) par rapport à l'orthèse de genou (51) et/ou par rapport à la potence (53) ; et/ou
- un capteur de force (56) qui surveille, via la potence (53) et l'élément de liaison (52), la force (N) introduite dans l'orthèse de genou (51).

# Fig. 1

Fixierung des Patienten (90)
am Kniemodul (50)

Festlegung der Ausgangsposition des Kniemoduls (50)

Manuelle Initialisierungsbewegung
eines Beins (92) des Patienten (90)

Aufzeichnung/Berechnung der
Trajektorie (T) des Kniegelenks (93)
(kinematisches Model)

Berechnung der
Länge (LO) des Oberschenkels (96)/
Position (YH/ZH) des Hüftgelenks(98)
(curve-fitting- Algorithmus)

Erstellung eine Modells

Speicherung/Anzeige
der Daten

Auslesen / weitere Nutzung
der Daten

Automatisierte Bewegung
eines Beins (92) des
Patienten (90)

Fig. 2a

90

93

97

96

T

$P_0$

98

92

Fig. 2b

z

$(y_3; z_3)$

$(y_2; z_2)$

$(y_1; z_1)$

y

F

Fig. 2c

z

$(y_3; z_3)$

$(y_2; z_2)$

$r = L_O$

$(y_1; z_1)$

T

y

$(y_H; z_H)$

F

Fig. 3

Fig. 4

## Fig. 5

## Fig. 6

Fig. 7a

Fig. 7b

Fig. 7c

# Fig. 8

# Fig. 9

# Fig. 10a

Fixierung des Patienten (90)
am Kniemodul (50)

Festlegung der Ausgangs-
Position des Kniemoduls (50)

Manuelle Initialisierungsbewegung
eines Beins (92) des Patienten (90)

Aufzeichnung/Berechnung der
Trajektorie (T) des Angriffspunkts (AP)
(kinematisches Model)

Berechnung der
Länge (Lo) zwischen Angriffspunkt (AP)
und Drehpunkt (DP) sowie
des Winkels θ_DP
(curve-fitting- Algorithmus)

Initialisierung eines Modells

Speicherung/Anzeige
der Daten

Auslesen / weitere Nutzung
der Daten

Automatisierte Bewegung
eines Beins (92) des
Patienten (90)

# Fig. 10b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2005334385 A
- JP H10258101 A
- WO 0061059 A1
- JP 2003225264 A
- CN 106621207 A
- DE 102015117596 B3
- WO 2017063639 A1
- JP H1176329 A
- DE 8514240 U1
- WO 2015127396 A1
- DE 10060466 A1